(19) Europäisches Patentamt
European Patent Office
Office européen des brevets

(11) EP 0 956 864 A1

(12) **EUROPEAN PATENT APPLICATION**
published in accordance with Art. 158(3) EPC

(43) Date of publication:
**17.11.1999 Bulletin 1999/46**

(21) Application number: **97946816.2**

(22) Date of filing: **03.12.1997**

(51) Int. Cl.$^6$: **A61K 38/50**, C12N 9/80, C12N 15/55

(86) International application number:
**PCT/JP97/04423**

(87) International publication number:
**WO 98/24473 (11.06.1998 Gazette 1998/23)**

(84) Designated Contracting States:
**AT BE CH DE DK ES FI FR GB GR IE IT LI LU MC NL PT SE**

(30) Priority: **03.12.1996 JP 32310596**

(71) Applicants:
- **KYOWA HAKKO KOGYO CO., LTD.**
  **Chiyoda-ku, Tokyo 100 (JP)**
- **Lee, Jong-Soo**
  **53359 Rheinbach (DE)**

(72) Inventors:
- **LEE, Jong-Soo**
  **D-53359 Rheinbach (DE)**
- **YANO, Keiichi**
  **Kawasaki-shi, Kanagawa 215 (JP)**
- **SATO, Mitsuo**
  **Houston, TX 77030 (US)**
- **MITSUI, Hideki**
  **Sunto-gun, Shizuoka 411 (JP)**
- **SUGIMOTO, Seiji**
  **Hachiohji-shi, Tokyo 192-03 (JP)**

(74) Representative:
**VOSSIUS & PARTNER**
**Postfach 86 07 67**
**81634 München (DE)**

(54) **TISSUE FIBROSIS INHIBITOR**

(57) Tissue fibrosis inhibiting agents comprising an arginine-degrading enzyme, a variant or a modified derivative thereof as an active ingredient. The tissue fibrosis inhibiting agents of the present invention are useful for preventing and treating tissue fibrosis and excellent in stability in blood and delivery to internal organs. The present invention also provides DNA molecules comprising the nucleotide sequence of SEQ ID NO: 7 or 9, DNA molecules which hybridize with the DNA molecules under stringent conditions and which encode a human arginase having a tissue fibrosis inhibiting activity, recombinant vectors containing the DNA molecules, transformants obtained by introducing the recombinant vectors into hosts, and a method of producing a human arginase, which comprises culturing the transformant in a medium to produce and accumulate a human arginase in the culture and recovering the human arginase from the culture.

FIG.3

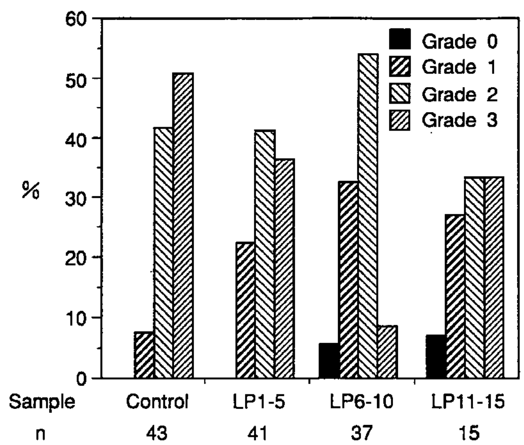




Printed by Xerox (UK) Business Services
2.16.7/3.6

## Description

### TECHNICAL FIELD

The present invention relates to tissue fibrosis inhibiting agents comprising an arginine-degrading enzyme, a variant or a modified derivative thereof as an active ingredient. The present invention also relates to DNA molecules encoding a human arginase, DNA molecules encoding a rat arginase, recombinant vectors containing the DNA molecules, transformants having the vectors introduced therein, and a method of producing arginase by using the transformant.

### BACKGROUND ART

It is known that the progress of the tissue fibrosis in liver (Handbuch der Speziellen Pathologie, 5, 243 (1930)), lung (Bull.Johns Hopkins Hosp., 74, 177, (1944)), kidney (J.Clin.Pathol., 34, 616, (1981)), pancreas (Zentralblatt fr Pathologie, 16, 903, (1905)), skin (Physiology and Pathology of the lymph vessel system.In:Lymph vessel system, Meessen,H. Springer, Berlin-Heidelberg-New York, 1972, p.239), and the like leads to the aggravation of diseases in these organs.

The course of a liver disease starts with acute hepatitis, which sometimes becomes chronic to develop into chronic hepatitis and then into hepatocirrhosis after many years. A fatal result will occur if hepatocirrhosis is complicated by hepatoma.

If the patient does not recover from acute hepatitis, the disease will develop into chronic hepatitis but then continuously injured hepatocytes are regenerated and the liver keeps its function at a sufficient level in many cases. In these conditions, the patient can enjoy and keep a normal life.

If hepatitis persists, macrophages are activated to englobe necrotic hepatocytes. On the other hand, fat-storing cells called "Ito cells" and other cells are transformed to fibroblasts and proliferated. These cells form a large amount of fibers to destroy a liver lobe. This is accompanied by hepatocyte proliferation to induce hepatocirrhosis so that the function of the liver decreases gradually to cause hepatic insufficiency or complications of hepatocirrhosis, which eventually lead to a death.

If hepatocirrhosis is prevented, it is expected that patients with chronic hepatitis who keep the function of liver at a sufficient level will survive for a long time. In addition, hepatoma mainly occurs as a complication of hepatocirrhosis, so the prevention of hepatocirrhosis leads to the prevention of hepatoma. Hence, the prevention of hepatocirrhosis is the most preferable treatment of patients with liver diseases.

Hepatitis is classified as viral hepatitis and alcohol-induced hepatitis. About 10-50% of acute viral hepatitis cases transform to chronic hepatitis. In the absence of a method for complete cure of chronic hepatitis, most patients with chronic hepatitis experience a progress of fibrosis into hepatocirrhosis. The fibrosis of the liver, which slowly progresses at the stage of chronic hepatitis, is mainly caused by the secretion of fibrins by proliferated fibroblasts which were transformed from Ito cells in the endothelia of sinusoids surrounding the portal veins of the liver. Hence, it is believed that Ito cells play an important role in the fibrosis of the liver. In alcohol-induced hepatitis, the formation of a fatty liver is followed by the progress of hepatic fibrosis at an early stage. As in the case of viral hepatitis, the fibrosis is mainly caused by proliferated fibroblasts that are transformed from Ito cells (Seminars in Liver Disease), Vol.10, p.30, 1990).

On the basis of the afore-mentioned findings, it is expected that if the transformation of Ito cells to fibroblasts and their proliferation can be inhibited, liver fibrosis can be inhibited to thereby reduce the onset of hepatocirrhosis and hepatoma (Molecular Medicine, 31, 2, (1994)).

Liver transplantation, in which the diseased liver is totally removed and replaced with a healthy liver at the terminal stage of a liver disease, may be mentioned as a method of first choice for the treatment of the liver disease. However, liver transplantation has the following problems: a) donors are by far fewer than patients who need liver transplantation, b) after transplantation, the patient is affected by side effects produced by an immunosuppressant administered against rejection, and c) transplantation is highly expensive.

A treatment of second choice is by preventing the infection with hepatitis viruses and killing them in the body if it is infected. However, hepatitis cannot be cured completely by this method because a new virus is produced, and sometimes, hepatitis viruses will undergo mutation.

Medicaments currently used for liver diseases include liver function ameliorating agents to compensate for the dysfunction of hepatocytes, anti-inflammatory agents to reduce inflammation, carcinostatic agents to cure hepatoma and the like. However, liver function ameliorating agents are only nosotropic and cannot stop the progress of liver diseases. As anti-inflammatory agents, steroids have been used but they are non-specific and produce strong side effects.

Other types of medicaments for liver diseases such as medicaments to promote the regeneration of necrotic hepatocytes or tissue fibrosis inhibiting agents to inhibit the transition to hepatocirrhosis are desired. Recently, hepatocyte growth factor (HGF) (Biochemical and Biophysical Res. Communications (hereinafter abbreviated to "B.B.R.C."), 122, 1450, (1984)) and hepatocyte growth substance (WO94/21678) have been reported as hepatocyte regeneration-promoting substances. Furthermore, an agent for inhibiting the synthesis of collagen that is a fiber constituting protein (Abstracts of Lectures in the 31st General Meeting of the Japan Liver Society, 1995, p.101) and an agent for inhibiting the crosslinkage of collagen fibrils (Abstracts of Lectures in the 31st General Meeting of the Japan Liver Society, 1995, p.62) are being studied as liver fibrosis inhibiting agents but their efficacy in clinical tests is not yet to be confirmed. Up to now, there has not been known any substance that inhibits the transformation of Ito cells to fibrin-secreting fibroblasts and/or the proliferation of Ito cells and fibroblasts.

Fibrosis in tissues of kidney, lung, pancreas or skin can also lead to a severe disease. However, no agents for inhibiting the tissue fibrosis of these organs have yet been known.

Arginase, arginine deiminase and the like are known as arginine-degrading enzymes.

Various kinds of arginase are known and they include: arginase playing a role in urea cycle in hepatocytes (J.Biol.Chem. 239, 3808 (1994)), arginase from a rat kidney and mammary gland (Biochemical Medicine & Metabolic Biology , 51, 156, (1994)), arginase from human erythrocytes (Biochem.J., 175, 449, (1978)), and arginase from mouse macrophages (B.B.R.C. 203, 1614, (1994)).

Regarding cDNA of arginase, only cDNAs of arginases from human and rat hepatocytes are known. There are several reports on the difference between these agrinases and others (Biological Chemistry Hoppe-Seyler,375,537,(1994)). However, cDNA of arginase is not clarified sufficiently. Up to now, an arginase gene from human hepatocytes (Proceedings of the National Academy of Science U.S.A. (hereinafter abbreviated to "Proc.Natl.Acad.Sci. U.S.A.") 84, 412, (1987) ; Nucleic Acids Research (hereinafter abbreviated to "Nucleic Acids Res."), 16, 8789, (1988)) and an arginase gene from rat hepatocytes (Proc.Natl.Acad.Sci. U.S.A. 84, 412, (1987) ; The Journal of Biological Chemistry (hereinafter abbreviated to "J.Biol.Chem.")262,6280,(1987);J.Biol.Chem.263,2245, (1988)) have been cloned.

There are reports on the biological activities of arginase administered in vivo and they include the inhibition of carbon monooxide production (FEBS Letters , 366, 127, (1995)), the inhibition of the proliferation of hepatoma cells (Cancer Biochemistry Biophysics, 13, (3), 171, (1993)), and the amelioration of liver function in combination with an anti-toxic component (French Patent, FR7722M, 1968). However, there is no report on the activities of arginase in inhibiting the proliferation of fibroblasts, inhibiting the transformation of Ito cells and their proliferation and inhibiting tissue fibrosis.

A variant of arginase in which a histidine residue is replaced with another amino acid residue is known. The study of the activity of the arginase variant when expressed reveals that a bivalent metal is bound at the position of a specific histidine, playing an important role in the arginase activity (Biochemistry, 33, 10652, (1994)). However, there is no report on an arginase variant that is excellent in arginase activity, enzyme stability and delivery to internal organs.

Regarding modified arginase derivatives, there are reports on an arginase derivative modified with polyethylene glycol (Cancer Biochemistry Biophysics, 13, 171, (1993)), a derivative in which arginase deiminase from mycoplasma is modified with polyethylene glycol (Japanese Journal of Cancer Research, 84, 1195, (1993)) and the like, and they are expected to have an improved stability in blood and lower antigenicity. However, there is no report on the study of a human arginase, a sugar-added derivative of arginase or a crosslinked arginase.

Although it is known that fibrosis in tissues of kidney, lung, pancreas or skin can lead to a severe disease, no agent for inhibiting the tissue fibrosis of these organs has yet known.

An object of the present invention is to inhibit the progress of tissue fibrosis with a substance or a derivative thereof which can prevent the fibrosis by inhibiting the transformation and proliferation of cells that are transformed to fibroblasts which play an important role in the tissue fibroblasts and by inhibiting the proliferation of the fibroblasts. Another object of the present invention is to provide tissue fibrosis inhibiting agents excellent in stability in blood and delivery to internal organs.

## DISCLOSURE OF INVENTION

The inventors found arginine-degrading enzymes, variants and modified derivatives thereof as substances capable of inhibiting the transformation of Ito cells to fibroblasts and their proliferation, as well as the proliferation of the fibroblasts and accomplished the present invention on the basis of this finding. Ito cells are known to play an important role in fibrosis of the liver which is one of the internal organs the tissue fibrosis of which can cause a severe disease. The present invention provides a tissue fibrosis inhibiting agent comprising an arginine-degrading enzyme, a variant or a modified derivative thereof as an active ingredient. Examples of the arginine-degrading enzyme include arginase, arginine deiminase and the like. The arginase may be of any origin such as human, rat or the like. These arginases may be extracted from an animal liver and subsequently purified. Alternatively, the arginase may be produced by genetic engineering techniques. Exemplary modified derivatives of the arginine-degrading enzyme include a polyethylene glycol-modified arginase and the like. The tissue fibrosis inhibiting agent of the present invention is particularly effective in inhibiting the tissue fibrosis of liver, kidney, lung, pancreas or skin.

The present invention also provides a DNA molecule comprising the nucleotide sequence of SEQ ID NO:7 and a DNA molecule which hybridizes with the nucleotide sequence of SEQ ID NO:7 under stringent conditions and which encodes a human arginase having a tissue fibrosis inhibiting activity. The present invention provides a recombinant vector containing the DNA molecule, a transformant obtained by introducing the recombinant vector into a host and a method of producing a human arginase, which comprises culturing the transformant in a medium to produce and accumulate a human arginase in the culture and recovering the human arginase from the culture.

Furthermore, the present invention provides a DNA molecule comprising nucleotide sequence of SEQ ID NO:9 and a DNA molecule which hybridizes with the nucleotide sequence of SEQ ID NO:9 under stringent conditions and which encodes a rat arginase having a tissue fibrosis inhibiting activity. The present invention also provides a recombinant vector containing the DNA molecule, a transformant obtained by introducing the recombinant vector into a host and a method of producing a rat arginase, which comprises culturing the transformant in a medium to produce and accumulate a rat arginase in the culture and recovering the rat arginase from the culture.

## BRIEF DESCRIPTION OF THE DRAWINGS

Fig. 1 shows the method for preparing liver perfusate.

Fig. 2 shows the criteria for judging liver fibrosing grades, wherein a, b, c and d are typical Sirius red F3BA stained images of the liver fibrosing grades 0, 1, 2 and 3, respectively.

Fig. 3 shows the results of measurement of liver fibrosis inhibiting activities when carbon tetrachloride was administered into rats to cause hepathopathy followed by in vivo administration of liver perfusate.

Fig. 4 shows the results of dilution assay in measuring Rat2 cell growth inhibiting activities of liver perfusates.

Fig. 5 shows the elution pattern of rat liver perfusates when purified by MonoS column chromatography.

Fig. 6 shows the elution pattern of the fraction containing Rat2 cell growth inhibiting activities eluted in the MonoS column chromatography when purified by Blue Cellulofine column chromatography.

Fig. 7 shows the Rat2 cell growth inhibiting activities of the fraction eluted in the Blue Cellulofine column chromatography.

Fig. 8 shows the elution pattern of the fraction containing Rat2 cell growth inhibiting activities eluted in the Blue Cellulofine column chromatography when purified by Red Sepharose CL-6B column chromatography.

Fig. 9 shows the inhibition of transformation to Ito cells by liver perfusate and purified arginase.

Fig. 10 represents the process for constructing plasmid pNS.

Fig. 11 represents the process for constructing plasmid pNS-hArg1.

Fig. 12 represents the process for constructing plasmid pNS-rArg1.

Fig. 13 shows the results of measurement of arginine concentrations in plasma taken 1, 3, 6 and 24 hours after the administration of PBS, rat arginase, or polyethylene glycol-modified rat arginase into rat.

Fig. 14 shows the results of measurement of arginine concentrations in plasma taken 1, 3, 7, 10 and 14 days after the administration of PBS or polyethylene glycol-modified rat arginase into rat.

Fig. 15 shows the results of measurement of arginase activities in plasma taken 1, 3, 6 and 24 hours after the administration of PBS, rat arginase, or PEG-modified rat arginase into rat.

Fig. 16 shows the results of measurement of arginase activities in plasma taken 1, 3, 7, 10 and 14 days after the administration of PBS or PEG-modified rat arginase into rat.

## BEST MODE FOR CARRYING OUT THE INVENTION

Arginine-degrading enzymes are enzymes which degrade or cleave arginine. The arginine-degrading enzymes include argninase and arginine deiminase. Arginase is an enzyme which hydrolyzes L-arginine into L-ornithine and urea and is widely distributed in liver and kidney of vertebrate animals, plant roots and embryos, and microorganisms. In human body, it is believed to play a role in nitrogen metabolism. Arginine deiminase is an enzyme which hydrolyzes L-arginine into L-citrulline and ammonia.

Argnine-degrading enzymes may be isolated and purified from a solution of perfusate of liver, kidney, lung, pancreas or skin, preferably liver, an extract obtained by homogenizing an organ, preferably liver, and removing insoluble matters by centrifugation, or serum. They may also be obtained by gene engineering methods.

Methods for preparing an arginine-degrading enzyme from a perfusate solution of an organ such as liver, kidney, lung, pancreas or skin, preferably liver, may comprise extracting an organ from a living body and perfusing it with an appropriate buffer. As an example, a method for preparing an arginine-degrading enzyme from liver will be described hereinbelow.

A liver may be removed according to a method described in Lee et al. [Surgery Forum (hereinafter abbreviated as "Surg. Forum"), 17, 220 (1966)]. Any other method may be used to remove liver so long as the liver per se and blood circulatory systems are not damaged. Liver is preferably removed by closing all blood circulatory systems of the liver tissues so that no perfusate can leak upon perfusion, except at the site where a perfusing catheter is inserted into the portal vein and the inferior venae cava above the liver.

Any method for perfusing liver may be used provided that no perfusate leaks upon perfusion. An excised rat liver is allowed to stand at preferably 10 to 40°C, more preferably 15 to 30°C, most preferably 20°C, for preferably 3 to 20 hours, more preferably 5 to 10 hours, most preferably 6 hours, and then perfused through portal vein with preferably 1 to 50 ml, more preferably 1 to 10 ml, most preferably 2 ml, per g liver, of Ringer solution at preferably 10 to 40°C, more preferably 15 to 30°C, most preferably 20°C. The flow rate may be 0.1 to 5 ml/min, preferably 0.5 to 2 ml/min, most preferably 1 ml/min, per g liver. In this manner, the liver is in vitro perfused. The perfusion is then repeated 1 to 20 times, preferably 5 times, with the same perfusing solution at intervals of 0 to 5 hours, preferably 30 minutes, and the perfusates are then recovered. Further, the same liver may be perfused with fresh Ringer solution in the same manner. A solution other than Ringer solution can be used in the perfusion to prepare liver perfusates, provided that it is a buffer which has a substantially neutral pH and an osmotic pressure similar to that of physiological saline. For example, Hank's balanced salt solution, Dulbecco's phosphate-buffered saline, Earle's balanced salt solution, Gey's balanced salt solution and Puck's saline may be used.

Arginine-degrading enzymes may be isolated and purified from an extract obtained by homogenization of an organ, preferably liver, in an appropriate aqueous solution and removal of insoluble matters by centrifugation, or from sera. For the isolation and purification, any separating operations such as solution extraction, fractionating precipitation with organic solvents, salting out, dialysis, centrifugation, ultrafiltration, ion exchange chromatography, gel filtration chromatography, hydrophobic chromatography, affinity chromatography, reverse phase chromatography, crystallization, and electrophoresis may be used alone or in combination. There is also a method using cation exchange carrier chromatography and the like to purify an arginine-degrading enzyme from a living body sample derived from rat and human liver: J. Biol. Chem., 239, 3808 (1964); and Arch. Biochem. Biophys., 320, 24 (1995).

Gene engineering technology may also be utilized to obtain arginine-degrading enzymes and variants of arginine-degrading enzymes.

The arginine-degrading enzymes and variants thereof may include, for example, a human arginase comprising the amino acid sequence of SEQ ID NO: 8, a rat arginase comprising the amino acid sequence of SEQ ID NO: 10, and those comprising an amino acid sequence in which one or more amino acids have been deleted from, replaced with or added to the amino acid sequence of the human or rat arginase and having a fibrosis inhibiting activity.

DNAs coding for an arginine-degrading enzyme or a variant thereof may include, for example, DNA comprising the nucleotide sequence of SEQ ID NO: 7 or 9, or DNAs hybridizing to the DNA under stringent conditions.

The DNA hybridizing to the nucleotide sequence of SEQ ID NO: 7 or 9 under stringent conditions is DNA in which one or more mutations, such as substitution, deletion, insertion and addition, have been introduced into the nucleotide sequence in such a manner that a protein encoded by the DNA maintains the fibrosis inhibiting activity. This DNA may be obtained using the DNA having the nucleotide sequence of SEQ ID NO: 7 or 9 as a probe by colony or plaque hybridization method: Molecular Cloning, A laboratoy mannual, 2nd Ed., Sambrook, Fritsch and Maniatis, Cold Spring Harbor Laboratory Press, 1989 (hereinafter referred to as "Molecular Cloning: A Laboratory Mannual, 2nd Ed."). More specifically, the DNA includes DNA identifiable by hybridization at 65°C with a probe which has been prepared, using a filter to which DNA from a colony or plaque is immobilized, in the presence of 0.7 to 1.0 M NaCl, and using DNA having the nucleotide sequence of SEQ ID NO: 7 or 9, by random primer method [which can be carried out according to the method described in Molecular Cloning: A Laboratory Mannual, 2nd Ed. or using a commercially available kit, for example, Random Primed DNA Labeling Kit from Boehringer Mannheim], followed by washing the filter in 0.1 - 2X SSC solution at 65°C (1X SSC solution means a solution comprising 150 mM NaCl and 15 mM sodium citrate).

Preparation and expression of DNA coding for an arginine-degrading enzyme or variant thereof can be carried out by the method as described in Molecular Cloning: A Laboratory Mannual, 2nd Ed., Current Protocols in Molecular Biology, Supplement 1-34, Greene Publishing Associates and Wiley-Interscience, 1987-1996 (hereinafter referred to as "Current Protocols in Molecular Biology, Supplement 1-34").

DNA coding for an arginine-degrading enzyme or variant thereof can be prepared in the following manner:

RNA is isolated from a human or rat tissue, preferably liver, or a cultured cell line, preferably one derived from liver, and used as a template to synthesize cDNA which is in turn incorporated into a suitable plasmid vector. The resultant recombinant vector is used to transform a host cell to prepare a cDNA library, from which clones of interest are selected to prepare DNA.

The processes for separating RNA from a human or rat tissue, preferably liver, or a cultured cell line, preferably one derived from liver, to prepare a cDNA library and for cloning can be carried out according to the methods described in Biochemistry, 18, 5294 (1977); JP A 2-227075; or commercially available experimental mannuals, such as "Gene Manupilation Experiments", ed. by Yasutaka Takagi, Kodan-sha; "Gene Mannual", ed. by Yasutaka Takagi, Kodan-sha; and "Molecular Cloning, A Laboratory Mannual", 2nd Ed.; or using a commercially available kit.

The methods for preparing total RNA from a human or rat tissue, preferably liver, or a cultured cell line, preferably one derived from liver may include thiocyanic acid guanidine-cesium trifluoroacetate method (Methods in Enzymol., 154, 3 (1987)), and the methods for preparing poly $(A)^+$ RNA from total RNA may include oligo (dT) immobilized cellulose column method (Molecular Cloning, A Laboratory Mannual, 2nd Ed.). The kits for preparing mRNA from a human or rat tissue, preferably liver, or a cultured cell line, preferably one derived from liver, may include Fast Track mRNA Isolation Kit (Invitrogen), and Quick Prep mRNA Purification Kit (Pharmacia).

The methods for synthesizing cDNA from poly $(A)^+$ RNA or mRNA may include the methods described in Molecular Cloning, A Laboratory Mannual, 2nd Ed. and Current Protocols in Molecular Biology, Supplement 1-34, and the methods using a commercially available kit, such as Super Script™ Preamplification System for First Strand cDNA Synthesis (Life Technologies).

The methods for preparing cDNA libraries may include the methods described in Molecular Cloning, A Laboratory Mannual, 2nd Ed. and Current Protocols in Molecular Biology, Supplement 1-34, and the methods using a commercially available kit, such as Super Script™ Plasmid System for cDNA Synthesis and Plasmid Cloning (Life Technologies) or ZAP-cDNA Synthesis Kit (Stratagene).

The DNA clone coding for an arginine-degrading enzyme may be selected from the cDNA library by using, for example, DNA coding for a rat arginase as reported by Ohtake et al. (J. Biol. Chem., 263, 2245 (1988)) or DNA coding for a human arginase as reported by Takiguchi et al. (Nucleic Acid Res., 16, 8789 (1988)) as a probe according to the colony or plaque hybridization method (Molecular Cloning, A Laboratory Mannual, 2nd Ed.).

Further, a primer may be prepared on the basis of the nucleotide sequence of the rat arginase reported by Ohtake et al. or the human arginase reported by Takiguchi et al., and DNA coding for an arginine-degrading enzyme may be prepared using cDNA or cDNA library synthesized from poly $(A)^+$ RNA or mRNA as a template by polymerase chain reaction (PCR) method (Molecular Cloning, A Laboratory Mannual, 2nd Ed.; Current Protocols in Molecular Biology, Supplement 1-34).

DNA for a varient of an arginine-degrading enzyme may be obtained on the basis of the DNA coding for an arginine-degrading enzyme obtained by the above described preparation method, according to the method as described in Molecular Cloning: A Laboratoty Mannual, 2nd. Ed. or Current Protocols in Molecular Biology, Supplement 1-34.

The cDNA clone selected in the above described method may be cut with appropriate restriction enzymes and cloned into a plasmid such as pBluescript KS(+) (Stratagene), and the nucleotide sequence of the DNA may then be determined by a nucleotide sequence analysis method usually used, for example, the dideoxy method (Sanger, et al., Proc. Natl. Acad. Sci. U.S.A., 74, 5463 (1977)). The analysis of nucleotide sequences may be carried out by using an automated nucleotide sequenser, for example, 373A DNA sequenser (Applied Biosystems).

The DNA coding for an arginine-degrading enzyme or a variant thereof obtained by the above described method may be inserted into a suitable vector downstream from the promoter thereof to construct a recombinant vector, which may then be introduced into a host cell to yield a transformant expressing the arginine-degrading enzyme or variant thereof.

The host may be any of bacteria, yeast, animal cells, insect cells and the like which can express a gene of interest. The bacteria may include microorganisms of the genus Escherichia, Serratia, Corynebacterium, Brevibacterium, Pseudomonas, Bacillus or the like, such as Escherichia coli, Bacillus subtilis, Bacillus amyloliquefaciens, Brevibacterium flavum, Brevibacterium lactofermentum, Corynebacterium glutamicum, and Microbacterium ammoniaphilum. The yeast may include Saccharomyces cerevisae, Schizosaccharomyces pombe, Kluyveromyces lactis, Trichosporon pullulans, and Schwanniomyces alluvius. The animal cells may include Namalwa cells which are human cells, COS cells which are monkey cells, and CHO cells which are chinese hamster cells. The insect cells may include Spodoptera frugiperda oocyte Sf9, Sf21 [Baculovirus Expression Vectors, A Laboratory Mannual, Oreilly, Miller and Luckow, W.H. Freeman and Company, New York, 1992 (hereinafter referred to as "Baculovirus Expression Vectors, A Laboratory Mannual)], and Trichoplusia ni egg cell Tn5 which is commercially available as High5 from Pharmingen.

The vector used to introduce the DNA coding for an arginine-degrading enzyme or variant thereof may be any vector into which the DNA coding for an arginine-degrading enzyme or variant thereof can be incorporated and which can be expressed in a host cell.

When a bacterium, such as Escherichia coli, is used as the host, the vector may preferably comprise a promoter, a ribosome binding sequence, the DNA of the present invention, a transcription terminating sequence, and an optional control sequence for the promoter.

An expression vector used may be, for example, pKYP10 (JP A 58-110600), pLSA1 (Agric. Biol. Chem., 53, 277 (1989)), pGEL1 (Proc. Natl. Acad. Sci. U.S.A., 82, 4306 (1985), or pNS.

The promoter used may be anyone which can be expressed in a host such as Escherichia coli. For example, promoters derived from Escherichia coli or phages, such as trp promoter (Ptrp), lac promoter (Plac), T7lac promoter,

$P_L$promoter, and $P_R$promoter, may be used. Artificially designed and modified promoters such as promoter comprising two Ptrp's in series (Ptrp x 2) and tac promoter may be used.

The ribosome binding sequence used preferably has a suitable distance between Shine-Dalgarno sequence (hereinafter abbreviated as "SD sequence") and the start codon adjusted to, for example, 6 to 18 bases.

If necessary, one or more bases in the nucleotide sequence of the DNA according to the present invention may be replaced or substituted in the recombinant vector of the present invention so that the sequence has optimum codons for the expression thereof in the host.

In the recombinant vector of the present invention, a transcription terminating sequence is not necessarily required to express the DNA of the present invention but preferably be disposed immediately downstream of the structural gene.

Methods for introducing a recombinant vector into a bacterium may be any one of the methods for introducing a DNA into a bacterium; for example, the method using calcium ion (Proc. Natl. Acad. Sci. U.S.A., 69, 2110-2114 (1972), and the protoplast method (JP A 63-2483942) may be used.

When yeast is used as a host, an expression vector such as YEp13 (ATCC 37115), YEp24 (ATCC 37051) or YCp50 (ATCC 37419) may be used.

Any promoters which can be expressed in a yeast may be used and include, for example, the promoter of genes relating to the glycolytic pathway such as hexose kinase genes, gal1 promoter, gal10 promoter, heat shock protein promoter, MF$\alpha$1 promoter, and CUP1 promoter.

Any methods for introducing DNA into a yeast may be used to introduce the recombinant vector into a yeast and include the electroporation method (Methods. Enzymol., 194, 182-187 (1990)), the spheroplast method (Proc. Natl. Acad. Sci. U.S.A., 84, 1929-1933 (1978)), and the lithium acetate method (J. Bacteriol., 153, 163-168 (1983)).

When an animal cell is used as a host, an expression vector, such as pAGE107 (JP A 3-22979; Cytotechnology, 3, 133 (1990)), pAS3-3 (JP A 2-227075), pAMoERC3Sc, pcDM8 (Nature, 329, 840 (1987)), pcDNAI/Amp, pcDNAI, both being manufactured by Funakoshi, Japan, may be used.

Any promoters which can be expressed in an animal cell may be used and include, for example, the promoter for IE (immediate early) gene of human cytomegalovirus (CMV), SV40 or metallothionein promoter. The enhancer for human CMV IE gene may also be used together with the promoter.

Any methods for introducing DNA into an animal cell may be used to introduce the recombinant vector into an animal cell and include, for example, the electroporation method (Cytotechnology, 3, 133 (1990)), the calcium phosphate method (JP A 2-227075), and the lipofection method (Proc. Natl. Acad. Sci. U.S.A., 84, 7413 (1987)).

When an insect cell is used as a host, a protein may be expressed by the method described in, for example, Current Protocols in Molecular Biology, Supplement 1-34; or Baculovirus Expression Vectors, A Laboratory Mannual. Thus, a vector for recombinant gene introduction and baculovirus described below are co-introduced into an insect cell to yield a recombinant virus in the supernatant of the insect cell culture and the recombinant virus is then infected into an insect cell to yield a transfected insect cell that expresses a protein of interest.

For example, pVL1392, pVL1393 or pBlueBacIII, all being manufactured by Invitrogen may be used as the vector for gene introduction.

Exemplary baculoviruses include, Autographa californica nuclear polyhedrosis virus which infects insects of the family Barathra.

Exemplary methods for co-introducing the above described vector for recombinant gene introduction and baculovirus into an insect cell to prepare a recombinant virus include the calcium phosphate method (JP A 2-227075), and the lipofection method (Proc. Natl. Acad. Sci. U.S.A., 84, 7413 (1987)).

With respect to methods for expression of genes, secretory production and fusion protein expression as well as direct expression have been developed and any one of these method can be used. For instance, the gene expression may be carried out according to the method as described in Molecular Cloning: A Laboratory Mannual, 2nd Ed.

When expressed in a yeast, animal cell or insect cell, there can be obtained an arginine-degrading enzyme or variant thereof to which a sugar or sugar chain has been added by the action of an exo- or endoglycosidase.

Any plasmid vectors into which the DNA coding for an arginine-degrading enzyme or variant thereof can be integrated may be used as plasmid vectors used to integrate DNA coding for an arginine-degrading enzyme or variant thereof and include, for example, pNS-hArg1 which is a plasmid vector for expression of an arginine-degrading enzyme, human arginase, and pNS-rArg1 which is a plasmid vector for expression of rat arginase. Escherichia coli BL21/pNS-hArg1 containing the plasmid vector pNS-hArg1 and Escherichia coli BL21/pNS-rArg1 containing the plasmid vector pNS-rArg1 were deposited on July 24, 1996 at National Institute of Bioscience and Technology, Ministry of International Trade and Industry, 1-3, Higashi 1-chome, Tsukuba-shi, Ibaraki-ken, Japan, under Accession Numbers FERM BP-5605 and FERM BP-5606, respectively.

The thus obtained transformant may be cultured in a medium to produce and accumulate an arginine-degrading enzyme or variant thereof in the culture. The arginine-degrading enzyme or variant thereof can be produced by recovering it from the culture.

The transformant of the present invention may be cultured in a medium according to the conventional procedures used to culture a host.

The medium for culturing the transformant obtained from a microorganism such as Escherichia coli or yeast as a host may be any of natural and synthetic media which contain a carbon source, a nitrogen source, mineral salts and the like utilizable by the microorganism and enable efficient cultivation of the transformant.

Exemplary carbon sources include carbohydrates such as glucose, fructose, sucrose, molasses, starch, and starch hydrolysates, organic acids such as acetic acid and propionic acid, and alcohols such as ethanol and propanol.

Exemplary nitrogen sources include ammonia; ammonium salts of inorganic or organic acids such as ammonium chloride, ammonium sulfate, ammonium acetate and ammonium phosphate; other nitrogen-containing compounds; as well as peptone, meat extract, yeast extract, corn steep liquor, casein hydrolysate, soybean cake and soybean cake hydrolysate, various fermented cells or their digests.

Exemplary mineral materials include potassium phosphate, dipotassium phosphate, magnesium phosphate, magnesium sulfate, sodium chloride, ferrous sulfate, manganese sulfate, copper sulfate and calcium carbonate.

Generally, the cultivation is carried out under aerobic conditions by, for example, shaking culture or culture with jar fermentor under aerobic conditions, at 15 to 40°C for 16 to 96 hours. The pH of the culture solution is maintained at 3.0 to 9.0 during the cultivation period. The pH may be adjusted with an inorganic or organic acid, an alkali solution, urea, calcium carbonate or ammonia.

If necessary, an antibiotic such as ampicillin or tetracycline may be added to the medium during the cultivation.

When a microorganism transformed with an expression vector comprising an inducible promoter as a promoter is cultured, an inducer may optionally be added to the medium. For example, isopropyl-β-D-thiogalactopyranoside (IPTG) may be added to the medium when a microorganism transformed with an expression vector comprising lac promoter is cultured. Indole-acrylic acid (IAA) may be added when a microorganism transformed with an expression vector comprising trp promoter is cultured.

The medium to be used to culture the transformant obtained from an animal cell as a host may be RPMI1640 medium, Eagle's MEM medium or a medium derived therefrom and supplemented with fetal bovine serum etc., these being conventionally used. Generally, the cultivation is carried out in the presence of 5% carbon dioxide at 35 to 37°C for 3 to 7 days. During the cultivation, an antibiotic such as kanamycin or penicillin may optionally be added to the culture medium.

When a transformant obtained from an insect cell as a host is cultured, TNM-FH medium (Pharmingen), Sf900IISFM (Life Technologies), and ExCell400 and ExCell405 (JRH Biosciences), which are conventionally used, are used. The cultivation is carried out at 25 to 30°C for 1 to 4 days in a culture medium optionally with an antibiotic (e.g. gentamycin) added.

When an arginine-degrading enzyme or a variant thereof is expressed in a soluble form in the cell or forms an insoluble body in the cell, the cultured cell is centrifuged, suspended in an aqueous buffer, and disrupted by ultrasonic or French press method. After centrifugation, the arginine-degrading enzyme or variant thereof is recovered from the supernatant.

When an insoluble body is formed in the cell, the insoluble body can be solubilized with a protein denaturing agent, and then diluted with or dialyzed against a solution which is free of protein denaturing agent or which contains a protein denaturing agent at such a low concentration that no protein is denatured, whereby the steric structure of the protein is regenerated.

When an arginine-degrading enzyme or a variant thereof or a derivative thereof such as one having a sugar or sugar chain added thereto is secreted out of the cell, the arginine-degrading enzyme or variant thereof or derivative thereof such as one having a sugar or sugar chain added thereto can be recovered from the supernatant after the cultivation. Isolation and purification may be carried out by one or any combination of the following separation operations; solvent extraction, fractionated precipitation with organic solvents, salting out, dialysis, centrifugation, ultrafiltration, ion exchange chromatography, gel filtration chromatography, hydrophobic chromatography, affinity chromatography, reverse phase chromatography, crystallization, electrophoresis.

The purified protein may be analyzed for the primary structure according to the method described in "Shin-Seikagaku Jikken Koza (New Biochemical Experimental Lectures), Vol. 1, Protein II, Primary Structure, ed. by Koichi Sentani, Tokyo Kagaku Dojin, Japan. For example, the N-terminal amino acid sequence can be determined by using an automated amino acid sequencer; or the protein is hydrolyzed with a protease such as trypsin and the resulting peptide fragments can be subjected to a mass spectrometer or an automated amino acid sequencer to determine the amino acid sequence. The determined amino acid sequence can be identified by a search for homology with amino acid sequences in protein databases such as PIR-International Protein Sequence Database (Protein Identification Resource) to identify.

The arginine-degrading enzyme or variant thereof can be modified with a sugar, sugar chain, polyvalent sugar complex or high molecular weight substance.

The sugar-, sugar chain- or polyvalent sugar complex-modified derivative can be produced as a glycoprotein by an animal, insect or yeast cell; or a sugar, sugar chain or polyvalent sugar complex may be preliminarily prepared and added enzymatically or chemically to the arginine-degrading enzyme or variant thereof.

The sugar and sugar chain may be prepared by chemical synthesis, degradation of polysaccharides, or release of a sugar chain from a glycoprotein optionally followed by purification of a sugar or sugar chain of a specific structure. The polyvalent sugar complex may be prepared by linking a sugar or sugar chain to a polyvalent carrier such as oligo- or polylysine: J. Drug. Targetting, 3, 111 (1995).

The resulting sugar, sugar chain or polyvalent sugar complex can also be added by means of a glycosyltransferase. Alternatively, it may be added by diazo coupling, isothiocyanate coupling, guanidination, or amidination method. The diazo and isothiocyanate coupling methods can be used for forming the linkage to benzene rings of tyrosine and phenylalanine in the protein, or to a imidazole group of histidine. The guanidination and amidination methods can be used for forming the linkage to an amino group at the side chain of lysine or to the N-terminal amino group of a protein: J. Drug. Targetting, 3, 111 (1995).

Any high molecular weight substances may be used which have low antigenicity and toxicity. For example, polyethylene glycols, dextrans, carboxy methyl celluloses, albumins, gamma-globulins, polyamino acids, styrene maleic acid copolymers and antibodies can be used: "Development of Pharmaceutical Products", Vol. 13, Drug Deliver Methods, Hirokawa Shoten, 1989, Japan; "Protein Hybrids - Most Advanced chemical modification" ed. by Yuji Inada, Kyoritsu Shuppan, 1987, Japan.

As methods for modification with the high molecular weight substance or polyvalent sugar complex, acid amide coupling, thioamide coupling, ester coupling, disulfide coupling, diazo coupling, alkylating, triazine ring coupling, isourea coupling, imide carboxylic acid coupling, and carbamic acid coupling methods have been developed ( "Protein Hybrids - Most Advanced chemical modification" ed. by Yuji Inada, Kyoritsu Shuppan, 1987, Japan; "Immobilized Biocatalysts" ed. by Ichiro Chibata, Kodansha, 1986, Japan; Motonori Ohno, Yuichi Kanaoka, Fumio Sakiyama and Hiroshi Maeda, "Chemical Modification of Proteins", Vol. 1-2, ed. by Ikuzo Uriya, Kensuke Shimura, Michinori Nakamura and Masaru Funatsu, Biochemical Experimental Methods 12, Gakkai Shuppan Center, 1981, Japan), and any one of the method may be used. Alternatively, the high molecular substance and arginine-degrading enzyme can be coupled through a linking agent by the above mentioned coupling method: "Immobilized Biocatalysts" ed. by Ichiro Chibata, Kodansha, 1986, Japan; Motonori Ohno, Yuichi Kanaoka, Fumio Sakiyama and Hiroshi Maeda, "Chemical Modification of Proteins", Vol. 1-2, ed. by Ikuzo Uriya, Kensuke Shimura, Michinori Nakamura and Masaru Funatsu, Biochemical Experimental Methods 12, Gakkai Shuppan Center, 1981, Japan.

Further, a high molecular weight substance that is modified with a sugar may also be coupled with the arginine-degrading enzyme or variant thereof.

The arginine-degarading enzyme or variant thereof may be intermolecularly cross-linked by any method, for example, by the method described in "Immobilized Biocatalysts" ed. by Ichi ro Chibata, Kodansha, 1986, Japan.

The arginine-degrading enzymes, variants thereof and modified derivatives thereof are effective in inhibiting fibrosis of living tissues. When the arginine-degrading enzyme, a variant thereof or a modified derivative thereof is used as a pharmaceutical drug, it can be administered by various routes such as injection, percutaneous mucosal administration, and oral administration in various dosage forms, depending on the administration route. Exemplary dosage forms include injections, such as intravenous, intramuscular, subcutaneous, and intradermal injections, suspension injections, in situ soluble injections, such as lyophilized injectable preparations, suppositories, intramucosal agents, such as intranasal agents, as well as oral agents, such as tablets, capsules, granules and suspensions. The preparations in these dosage forms may be formulated by any conventionally known method and contain pharmaceutically acceptable preservatives, stabilizers, antioxidants, excipients, binders, disintegrators, wetting agents, lubricants, colorants, aromatics, flavors, coatings, suspending agents, emulsifiers, solubilizing agents, buffers, isotonicities, plasticizers, surfactants, soothing agents, and the like, depending on their purposes.

Exemplary pharmaceutical carriers to be used include water, distilled water for injection, physiological saline, glucose, fructose, sucrose, mannitol, lactose, starch, cellulose, methyl cellulose, carboxy methyl cellulose, hydroxypropyl cellulose, glycerin, mannitol, xylitol, sorbitol, glucuronic acid, hyaluronic acid, heparin, chitin, chitosan, glycine, alanine, proline, serine, cysteine, aspartic acid, glutamic acid, lysine, arginine, human serum albumin, human serum globulin, collagen, gelatin, algic acid, talc, sodium citrate, calcium carbonate, calcium hydrogen phosphate, magnesium stearate, urea, silicone resin, sorbitan fatty acid ester, glycerin fatty acid ester, ascorbic acid, $\alpha$-tocopherol and the like.

A dose may vary with the administration route, and age, body weight and symptom of a patient; usually 0.01 $\mu$g to 10 g/60 kg per day may be suitable.

The fibroblast growth inhibiting activity of the arginine-degrading enzyme, a variant thereof and a modified derivative thereof can be assayed, preferably by a method using those cells which are considered to exhibit similar behavior to that of Ito cells or fibroblasts transformed from the Ito cells in liver. A procedure of assay using rat fetal fibroblast cells or rat fibroblast cell line Rat2 will be explained below.

Rat fetal fibroblasts are prepared in the following manner: About 10 days after mating, a fetus is aseptically removed from a pregnant SD rat, and the head and limb are removed. The remainder is washed and cut into small pieces with scissors. Fibroblasts are recovered from the tissue pieces. Further, the fibroblasts are centrifuged to remove erythrocytes. After adding a medium, the resulting cell suspension of 0.5 to 20 x $10^5$ cells/ml, preferably 2 x $10^5$ cells/ml is incubated at 37°C under 5% carbon dioxide conditions. The cells are subcultured at intervals of 1 to 7 days, preferably 3 to 5 days, depending on the degree of cell growth.

The fibroblasts subcultured through 2 - 10 cycles, preferably 3 - 6 cycles are used to assay the fibroblast growth inhibiting activity. After the culture medium is removed from the culture vessel, the attached cells are washed with an isotonic neutral buffer such as PBS at 10 to 40°C, preferably 37°C, and treated with Dispase grade II solution (Boehringer Mannheim) to isolate the cells. Then, a medium is added to prepare a cell suspension.

Any vessel may be used in assay of the fibroblast growth inhibiting activity; preferably, Terasaki plate is used. An example of assay using Terasaki plate will be shown below. To one well, 100 to 500 cells, preferably 300 cells, are added and 1 to 50 µl, preferably 5 µl, of a sample solution is added thereto. In a control experiment, an isotonic neutral buffer such as Ringer solution is added instead of the sample solution. The sample solution is incubated under 5% carbon dioxide conditions for 24 to 120 hours, preferably 72 hours. After the cultivation, the Terasaki plate is washed with an isotonic neutral buffer such as PBS and the cells are fixed with methanol. After drying, the cells are stained with Giemsa solution and the number of cells is counted. The following equation is used to calculate the growth inhibition:

$$IR (\%) = \{(C-T)/C\} \times 100$$

wherein IR is the growth inhibition, C is the number of cells when Ringer solution is added instead of a sample solution, and T is the number of cells when a sample solution is added.

The assay of growth inhibiting activity using the rat fetal fibroblast as above mentioned has an insufficient sensitivity and needs complicated operations; therefore, a higher sensitive assay of the growth inhibiting activity using rat fibroblasts, Rat2 cells, was established. This assay will be described hereinbelow.

Rat fibroblast cell line Rat2 (ATCC CRL-1764) was cultured in Eagle MEM medium containing 10% bovine serum (Science, 130, 432 (1959)) in the presence of 5% carbon dioxide at 37°C.

The number of Rat2 cells can be measured by any method which can be used for quantitative determination of cell growth. Exemplary methods include the MTT method established by Mosmann (J. Immunol. Methods, 65, 55 (1983)), the method of measuring the intake of a compound labelled with a radioisotope such as tritium thymidine within cells, the method of counting the number of cells, or the method of measuring a specific enzymatic activity in cells. Procedures for quantitatively determining the number of cells by the MTT method and assaying the cell growth inhibiting activity of a sample will be described herein.

Rat2 cells grown to a confluent state are washed and digested to exfoliate individual cells from the attached surface. At this stage, Eagle MEM medium containing 2 to 20%, preferably 10%, of bovine serum is added thereto to terminate the enzymatic digestive reaction of actinase E. After centrifugation, Eagle MEM medium containing 2 to 20%, preferably 10%, of bovine serum is used to prepare 0.2 to 20 x $10^5$ cells/ml, preferably 1.2 x $10^5$ cells/ml of a cell suspension and the cell suspension is distributed in 96-well cell culture plates at 25 to 200 µl/well, preferably 100 µl/well. A sample is added to Rat2 cells and incubated in the presence of 5% carbon dioxide at 37°C for 24 to 120 hours, preferably 72 hours.

Thereafter, PBS containing 1 to 10 mg/ml, preferably 5 mg/ml, of MTT reagent is added at 1 to 100 µl/well, preferably 10 µl/well, and further incubated for 1 to 8 hours, preferably 4 hours. Then, an isopropanol solution containing 0.01 to 0.5 N, preferably 0.04 N, hydrochloric acid is added at 5 to 200 µl/well, preferably 150 µl/well, to dissolve the produced formazan dye. The absorbance of the dissolved formazan dye at 540 nm is measured to thereby quantitatively determine the number of cells.

Alternatively, an assay can also be done in a morphological methodology using the transformation of Ito cells to fibroblasts as an index.

The Ito cells can be prepared by the method described by Kawada et al. (Eur. J. Biochem., 213, 815 (1993)) but any method which can be used to isolate Ito cells efficiently may be used.

Ito cells are suspended with Dulbecco MEM medium [(Virology, 8, 396 (1959)] containing 2 to 20%, preferably 10%, of bovine serum to 0.5 to 10 x $10^5$ cells per well, preferably 1.7 x $10^5$ cells per well, distributed in 12-well cell culture plates at 1 to 4 ml/well, preferably 2 ml/well, and incubated in the presence of 5% carbon dioxide at 37°C for 2 days. After the medium is removed, a medium containing a sample is added at 1 to 4 ml/well, preferably 2 ml/well, and further incubated in the presence of 5% carbon dioxide at 37°C for 2 to 6 days, preferably 4 days. Again the medium is removed, the medium containing the sample is added at 2 ml/well, and further incubated for 3 to 10 days, preferably 7 days. During the incubation period, the morphological change of Ito cells was observed by an optical microscope. Any other methods which can be used to observe the transformation into fibroblasts and their growth may be used.

In another method, the liver fibrosis inhibiting activity can also be assayed by using an animal model of hepatocirrhosis.

As the animal model of hepatocirrhosis, a carbon tetrachloride model can be made according to Lee's method (Therapiewoche, 33, 51 (1983)) as shown below.

A hepatocirrhosis inducer to be used is carbon tetrachloride which has been diluted with olive oil at 10 to 70% (v/v), preferably 50%. The carbon tetrachloride is sterilized with a 0.45 μm filter membrane, the olive oil has been sealed in a Medium bottle, and the instruments used upon preparation of pharmaceuticals has been subjected to high pressure steam sterilization (121°C, 1.2 atoms, 20 minutes). Upon dilution, each reagent is taken by a glass syringe, two syringes containing the respective reagents are connected with a connecting needle, and pumping is done 1 to 100 times, preferably 5 to 60 times, most preferably 50 times, to yield a homogeneous solution. The carbon tetrachloride solution is prepared in situ or once a week, sealed in a brown vial bottle and stored in a cool and dark place.

Animals to be used are SD male rats (body weight of 150 to 300 g, preferably 200 to 250 g) purchased. These animals are bred about 1 to 2 weeks and divided into a group to which a sample to be tested is administered and a control group.

After an inhalation anesthesia treatment of the rats with diethyl ether, the carbon tetrachloride solution is injected into the femurs of rats under anesthesia at 0.5 to 2 ml, preferably 0.5 to 1.0 ml, per kg of body weight by means of a plastic syringe, at intervals of 1 to 7 times a week, preferably 2 to 3 times a week, for 4 to 8 weeks, preferably 6 weeks.

The prepared animal model of hepatocirrhosis is used to assay the liver fibrosis inhibiting activity. After rats are preliminarily subjected to an inhalation anesthesia treatment with diethyl ether, 0.1 to 5 ml, preferably 1 to 3 ml, most preferably 2 ml, per rat of a sample to be tested or Ringer solution as a control is injected into the penis veniplexes by means of a disposable plastic syringe. At the day at which carbon tetrachloride is to be injected, the sample or Ringer solution is injected following the injection of carbon tetrachloride. The administration may be continued from 2 - 4 weeks, preferably 3 weeks to 4 - 8 weeks, preferably 6 weeks after the start of administration of carbon tetrachloride. The intervals of administration may be once per 1 to 7 days, preferably 1 to 2 days, most preferably every day.

After the administration has completed, the rat was anatomized to remove the liver. The tissue specimens are fixed with formalin and stained with Hematoxylin Eosin (HE) and Fast-green Sirius red (FS) or Sirius-red F3BA in order to specifically stain both collagenous and elastic fibers. The fibers are stained red with Sirius red. Thus, depending on the stage of fibrosis, the liver fibrosing grade of each individual is estimated by the following 4-grade criterion:

Grade 0: No production of new collagen is observed;
Grade 1: The production of collagen starts;
Grade 2: The production of collagen is completed in the portal regions; and
Grade 3: Hepatocirrhosis occurs.

The present invention will be explained in more detail by the following examples. These examples are given only for illustration but not intended to limit the scope of the present invention.

Example 1

It will be hereinbelow shown that materials which were obtained from a perfusate of liver, kidney, lung, pancreas or skin, preferably liver, an extract obtained by homogenization and centrifugation of insoluble materials, or serum, and which have liver fibrosis inhibiting activity, fibroblast growth inhibiting activity and Ito cells transformation inhibiting activity, are argnine-degrading enzymes.

1. Preparation of liver perfusate

Livers were excised according to the method of Lee et al. (Surg. Forum, 17, 220 (1966)).

Male SD rats (Zentraltierzucht) with a body weight of 250 to 270 g were anesthetized with ether. After anesthsia, the rat aodomen was washed with 70% ethanol to disinfect well. The rat was transferred into a clean bench, and the skin in the hypogastrium was incised along the median line. Further, the abdominal cavity portion was skined up to the vicinity of the back. The abdominal cavity was also incised along the median line like the skin. Once viscera were exposed, the intestine was put aside to the right and the liver was put aside to the diaphragm with a wet gauze, whereby the portal vein was fully exposed. A sutural thread was placed around the portal vein to form a loop. Like the portal vein, a lood was made around the inferior venae cava below the liver with a sutural thread. The portal vein was incised in half vertically at the peripheral side using scissors and a catheter into which Ringer solution had been introduced at 20°C was inserted. The portal vein was strongly tied with a sutural thread to fix the catheter. Immediately thereafter, the inferior venae cava below the liver was cut to dehematize while simultaneously the blood in the liver was washed a way by perfusing 30 ml of Ringer solution at 20°C at a flow rate of 10 ml per minute. Then, the inferior venae cava below the liver was closed by tying strongly with a sutural thread. The inferior venae cava above the liver was incised and a separately prepared catheter was inserted thereinto. The inserted catheter was fixed by strongly tying with a sutural thread.

After a route for liver persufion was thus made, all other blood vessels linked to the liver were closed with sutural threads and the liver was removed out carefully so that the liver was not damaged. The above procedures were summarized in Fig. 1.

Subsequently, a disorder was caused in hepatocytes by ischemia. Livers can generally be stored at 20°C for 2 to 3 hours; therefore, the liver was allowed to stand for 6 hours which was double the time. The reason therefore is that the fibroblast growth inhibiting activity was highest at 6 hours of ischemia as shown in 4 below. The liver is perfused through the portal vein with Ringer solution of 2 ml per g of liver at 20°C. The flow rate was 1 ml/min per g of liver which corresponded to the blood flow rate in the living liver. According to the above described perfusion method, perfusion was five times repeated with the same perfusate at intervals of 30 minutes. The collected perfusates were designated as LP1 to LP5. Further, the same liver was perfused five times with fresh Ringer solution of 2 ml per g of liver at intervals of 30 minutes and the collected perfusates were designated as LP6 to LP10. Thereafter, the same liver was further perfused five times with fresh Ringer solution of 2 ml per g of liver at an interval of 30 minutes and the collected perfusates were designated as LP11 to LP15. With these liver perfusates LP1 to LP5, LP6 to LP10, and LP11 to LP15, the liver fibrosis inhibiting activity, fibroblast growth inhibiting activity, and Ito cell transformation inhibiting activity were assayed according to the method as described in 2 below, the method as described in 3 below, and the method as described in 7 below, respectively.

2. Assay of liver fibrosis inhibiting activity in animal model of hepatocirrhosis

(1) Preparation of animal model of hepatocirrhosis

A hepatocirrhosis inducer used was carbon tetrachloride (Merck) which had been diluted with olive oil (DAB9) at 50% (v/v). The carbon tetrachloride was sterilized with 0.45 μm filter membrane (Costar), and the olive oil was sealed in a Medium bottle and both of them were subjected to high pressure steam sterilization (121°C, 1.2 atoms, 20 minutes). Also, the instruments used upon preparation of the below mentioned pharmaceuticals were all subjected to high pressure steam sterilization. Upon dilution, each reagent was taken by a 20 ml glass syringe with a Luer lock, two syringes containing the reagent were connected with a connecting needle, and pumping was repeated 50 times to yield a homogeneous solution. The inducer was prepared once a week, sealed in a brown vial bottle and stored in a cool and dark place.

Male SD rats (Zentraltierzucht, body weight of 220 to 240 g) were purchased, and divided into a group to which the liver perfusate was administered and a group to which Ringer solution was administerd after about 1 week breeding wherein the group to which the liver perfusates LP1 to LP5 were administered consisted of 41 rats, the group to which the liver perfusates LP6 to LP10 were administered consisted of 37 rats, the group to which the liver perfusates LP11 to LP15 were administered consisted of 15 rats, and the group to which the Ringer solution was administered consisted of 43 rats.

After inhalation anesthesia with diethyl ether, 1 ml of carbon tetrachloride preparation was intramuscularly administered to the femur under anesthesia at 0.5 ml of carbon tetrachloride per kg of body weight by means of a 1 ml plastic syringe with a Luer lock (Braun Melsungen AG). The administration was done twice a week, on Thursday and Friday every week, for 6 weeks.

(2) Methods of administration of liver perfusate and Ringer solution

After the rats were treated by diethyl ether inhalation anesthesia preliminarily prior to the administration (or after the administration on Thursday and Friday when carbon tetrachloride was to be administered), 2 ml of the liver perfuaste or Ringer solution per rat was administered into penis veniplex by means of a 2.0 ml disposable plastic syringe (Braun Melsungen AG). The administration was done every day on 3rd to 6th weeks from the start of administration of carbon tetrachloride.

(3) Necrotomy and treatment

On 3rd day from the last administration of carbon tetrachloride, the rats were sacrificed and the liver was removed out by dissection of the rats. Tissue specimens of 2 to 3 mm in thickness from each lobe of the liver were fixed with formalin. After one week from the fixation, the pathological tissue specimens of the liver were stained with Hematoxylin Eosin and Sirius red F3BA staining method to specifically stain both collagenous and elastic fibers. The stages of fibrosis were classified into following 4 grades:

Grade 0: No production of new collagen to observed;
Grade 1: The production of collagen starts;

Grade 2: The production of collagen is completed in the portal regions; and

Grade 3: Hepatocirrhosis occurs.

Fig. 2 shows typical stained images. In Fig. 2, a, b, c and d are typical Sirius red F3BA stained images of liver fibrosis grades 0, 1, 2 and 3, respectively. These were used as standards of liver fibrosis grades to judge the liver fibrosis grade of each individual.

Fig. 3 shows liver fibrosis grades of the administration group of liver perfusates LP1 to LP5, LP6 to LP10 and LP11 to LP15 and the administration group of Ringer solution. As compared with the stage (grade 3) of hepatocirrhosis of the control group to which Ringer solution was administered, the hepatocirrhosis is strongly inhibited in the LP6 to LP10 administration group (compare bars of grade 3). Thus, it was revealed that LP6 to LP10 have a strong effect on prevention of hepatocirrhosis.

3. Assay of rat fetal fibroblast growth inhibiting activity (1) Preparation of rat fetal fibroblast

Fibroblasts were taken from 5 fetuses of one pregnant SD rat at about 10 days after mating. The abdomen of the pregnant rat was disinfected with 70% ethanol and the rat was etherized. All subsequent procedures were aseptically

carried out. After the celiotomy, the uterus was removed out and placed in a Petri dish. In a clean bench, at least 5 fetuses were removed from the uterus and the head and limb were removed. The remainder was washed twice with PBS (Flow Laboratories) and cut into small pieces with scissors. The tissue specimens were placed in an Erlenmeyer flask and 10 ml of a trypsin solution (0.25%, Biochrom) was added. After the flask was spun in a magnet tube (IKA-combimag, Rco Janke & Kunkel) at 37°C for 5 minutes, the resultant supernatant was transferred to a beaker and 20 ml of a medium (MEM medium containing 10% fetal bovine serum (Flow Laboratories), 2 mM L-glutamine (Boehringer, Mannheim), 1% penicillin-streptomycin (Hoechst, AG)) was added to stop the trypsin treatment. To the remaining tissue specimens, the trypsin solution was again added and the same procedures were repeated 4 times to collect as many fibroblasts as possible.

(2) Primary culture

The thus prepared cell suspension was centrifuged at 1,000 rpm for 10 minutes and the supernatant was removed out. Thereafter, the residue was washed twice with PBS at 37°C to remove erythrocytes. After adding a medium to the remaining cells to prepare a cell suspension of 2 x $10^5$ cells/ml, 10 ml of the cell suspension was placed in a culture vessel (Falcon, 3013E, 25 $mm^2$ style, Becton Dickiness) and incubated at 37°C under 5% carbon dioxide conditions.

(3) Subculture of fibroblast

After the culture solution in the culture vessel was removed out and the attached cells were washed three times with 6 ml of PBS at 37°C, 5 ml of the trypsin solution was added to soak the cell layer. After 5 minutes, 10 ml of the medium was added to stop the trypsin treatment. The resulting cell suspension was centrifuged at 1,000 rpm. The supernatant was removed out, and the medium was added to the remaining cells to prepare a cell suspension of 2 x $10^5$ cells/ml. Ten milliliter of the cell suspension was placed in a culture vessel and cultured at 37°C under 5% carbon dioxide conditions. The medium was changed every 3 days and the cells were subcultured once per 3 to 5 days, depending on the degree of cell growth.

(4) Assay of fibroblast cell growth inhibiting activity

To assay the fibroblast growth inhibiting activity, fibroblasts subcultured through 3 to 6 cycles were used. After the culture solution was removed from the culture vessel, the attached cells were washed three times with 6 ml of PBS at 37°C. Five ml of Dispase grade II solution (Boehringer Mannheim) was added to soak the cell layer. Once the cells were isolated after 10 to 30 minutes, 10 ml of the medium was added to stop the Dispase treatment. A cell suspension was prepared and centrifuged at 1,000 rpm for 10 minutes. The supernatant was removed out, and the medium was added to the remaining cells to prepare a cell suspension of 6 x $10^4$ cells/ml. In this step, it was confirmed by Trypan Blue staining that the proportion of living cells was 97% or higher.

A Terasaki plate was used to assay the growth inhibiting activity. In one well, 300 cells per 5 μl medium were placed and 5 μl of a sample solution was added thereto. Samples to be tested were the liver perfusates LP1 to LP5, LP6 to LP10, LP11 to LP15 and solutions thereof diluted with Ringer solution to percent dilutions as shown in Table 1. In a control experiment, Ringer solution was added instead of the sample solution. The reaction mixture was incubated under 5% carbon dioxide conditions for 72 hours.

(5) Fixation and staining of cells

After 72 hours incubation, the Terasaki plate was washed twice with PBS and the cells were fixed with methanol for 10 minutes. After drying in air for 20 minutes, the fibroblasts were stained with Giemsa solution diluted 10 times with distilled water and the number of cells was counted. The following equation was used to calculate the growth inhibition:

$$IR (\%) = \{(C-T)/C\} \times 100$$

wherein IR is the growth inhibition rate, C is the number of cells when Ringer solution is added instead of a sample solution, and T is the number of cells when a sample solution is added.

The results are shown in Table 1.

Table 1

| IR (%) | | | |
|---|---|---|---|
| Dilution (%) | LP1-5 | LP6-10 | LP11-15 |
| 2.5 | 51.40±8.14 | 72.22±6.72 | 49.66±6.09 |
| 5 | 90.86±4.08 | 95.07±4.00 | 75.94±5.96 |
| 10 | 99.77±0.23 | 100.00±0.00 | 87.94±5.48 |
| 20 | 100.00±0.00 | 100.00±0.00 | 99.95±0.51 |
| 40 | 100.00±0.00 | 100.00±0.00 | 100.00±0.00 |
| 100 | 100.00±0.00 | 100.00±0.00 | 100.00±0.00 |

The liver perfusates LP1 to LP5, LP6 to LP10 and LP11 to LP15 all showed rat fetus-derived fibroblast growth inhibiting activity. Further, when the perfusates were diluted to 100%, 40%, 20%, 10%, 5% and 2.5% and then the fibroblast growth inhibiting activity was assayed, LP6-10 showed the strongest growth inhibiting activity. This indicates that there is a relationship between the in vitro rat fetus-derived fibroblast growth inhibiting activity and the in vivo liver fibrosis inhibiting activity and suggests that it is reasonable to search liver fibrosis inhibiting factors by using the fibroblast growth inhibiting activity as an indicator.

4. Investigation of ischemic period upon preparation of liver perfusates

The optimum ischemic period for recovery of fibroblast growth inhibiting activities was investigated. As in 1 of Example 1, the liver of male SD rats of 250 to 270 g in body weight was removed out and allowed to stand at 20°C for 0, 3, 6 or 9 hours. Then Ringer solution was used to prepare LP1 to LP5, LP6 to LP10 and LP11 to LP15 and the equation as shown in 3(5) of Example 1 was used to calculate the fetal fibroblast growth inhibiting activity (IRs) described in 3 of Example 1. The results are shown in Table 2.

Table 2

| | | IR (%) | | | |
|---|---|---|---|---|---|
| Sample | Dilution (%) | Ischemic period (hr) | | | |
| | | 0 | 3 | 6 | 9 |
| LP1-5 | 2.5 | 0.0±0.0 | 21.0±5.0 | 51.4±8.1 | 1.8±1.2 |
| | 5.0 | 0.3±0.4 | 41.2±6.6 | 90.9±4.1 | 20.4±4.8 |
| LP6-10 | 2.5 | 0.0±0.0 | 26.4±5.4 | 72.2±6.7 | 22.1±5.3 |
| | 5.0 | 0.9±0.6 | 50.5±7.6 | 95.1±4.0 | 89.1±3.1 |

Table 2 (continued)

| | | IR (%) | | | |
|---|---|---|---|---|---|
| Sample | Dilution (%) | Ischemic period (hr) | | | |
| | | 0 | 3 | 6 | 9 |
| LP11-15 | 2.5 | 0.0±0.0 | 13.8±4.8 | 49.7±6.1 | 2.1±1.4 |
| | 5.0 | 0.3±0.3 | 38.1±8.7 | 75.9±6.0 | 14.2±3.6 |

The perfusate of the liver without ischemia and therefore without injury had no inhibiting activity. On the other hand, the largest amount of a fraction having the fibroblast growth inhibiting activity was recovered from the liver perfusate after 6 hours ischemia. Accordingly, the period of ischemia was to be set at 6 hours.

5. Assay of cell growth inhibiting activity of liver perfusate in rat fibroblast cell line Rat2

In assaying the cell growth inhibiting activity in the liver perfusate, Rat2 cells, which are rat fibroblast cells, were also used in addition to the rat fetal fibroblast cells and their growth inhibition was used as an index.

(1) Preparation of fibroblast

Rat2 cells (ATCC CRL-1764) were cultured in Eagle MEM medium (Science, 130, 432 (1959)) containing 10% bovine serum (Hyclone Laboratories) in the presence of 5% carbon dioxide at 37°C.

(2) Assay of cell growth inhibiting activity in liver perfusate

The number of Rat2 cells may be measured by any method which can be used to determine cell growth in a quantitative manner. The number of cells was quantitatively determined by the MTT method herein to measure the cell growth inhibiting activity in the liver perfusates LP6 to LP10.

Rat2 cells grown to a confluent state were washed with PBS and PBS containing 0.001% Actinase E (Kaken Seiyaku Kabushiki Kaisha) and 0.02% EDTA was added. Once the digestion proceeded and individual cells were exfoliated from the attached surface, Eagle MEM medium containing 10% of bovine serum was added thereto to stop the enzymatic digestive reaction of Actinase E. After centrifugation, Eagle MEM medium containing 10% bovine serum was used to adjust the cell density to $1.2 \times 10^5$ cells/ml and the cell suspension was seeded in a 96-well cell culture plate at 100 μl/well. To the wells to which Rat2 cells were seeded, the liver perfusates LP6 to LP10 were added and the cells were cultured in the presence of 5% carbon dioxide at 37°C for 72 hours.

After 72 hours cultivation of Rat2 cells, PBS containing 5 mg/ml of MTT reagent was added at 10 μl/well, and further cultured for 4 hours. Then, an isopropanol solution containing 0.04 N hydrochloric acid was added at 150 μl/well to dissolve the produced formazan dye. The absorbance of the dissolved formazan dye at 540 nm was measured to quantitatively determine the number of cells.

Fig. 4 shows the results of the measurement by the above mentioned method as to the effects of the liver perfusates LP6 to LP10 prepared in 1 of Example 1 on the cell growth of Rat2 cells. The prepared liver perfusates LP6 to LP10 inhibited the growth of Rat2 cells, which were fibroblasts, in a concentration-dependent manner. IC50 was about 1%.

6. Isolation and structural analysis of Rat2 cell growth inhibitory active substances from liver perfusates LP6 to LP10

Isolation of the Rat2 cell growth inhibitory active substances from the liver perfusates LP6 to LP10 was carried out in the following manner:

Forty (40) ml of the liver perfusates LP6 to LP10 obtained in 1 of Example 1 were subjected to dialysis against 1 L of 10 mM phosphate buffer (pH 7.0) using a seamless cellulose membrane (Viskase Sales Corp.). The dialysate was filtered through MILLEX GV (Millipore) and subjected to Mono S column chromatography under the following conditions:

Condition 1:

| | |
|---|---|
| column: | Mono S (10/10) (10x100 mm, Pharmacia); |
| eluent: | A solution (10 mM phosphate buffer, pH 7.0); |
| | B solution(10 mM phosphate buffer, pH 7.0, 1.0 M sodium chloride); |
| flow rate: | 2 ml/min; |
| elution: | B solution, 0% - 30% linear concentration gradient, 144 ml. |

The elution of protein was monitored by measuring absorbance at 280 nm. Effluents were collected by a fraction collector and Rat2 cell growth inhibiting activity of each fraction was assayed by the method as described in 5 of Example 1. The results are shown in Fig. 5. The gradient elution was commenced at the point of 85 ml in the abscissa. The hatched box represents the main fractions of Rat2 cell growth inhibiting activity.

The fractions represented by the hatched bar in Fig. 5 which showed the growth inhibiting activity were further subjected to chromatography using a dye affinity carrier Blue Cellulofine (Chisso)-packed column under the following conditions:

Condition 2:

| | |
|---|---|
| column: | Blue Cellulofine-packed Column (10x100 mm); |
| eluent: | A solution(20 mM phosphate buffer, pH 7.5, 0.1 mM nickel chloride); |
| | B solution (20 mM phosphate buffer, pH 7.5, 1.0 M sodium chloride. 0.1 mM nickel chloride); |
| | C solution(20 mM phosphate buffer, pH 7.5, 1.0 M sodium chloride, 60% ethylene glycol, 0.1 mM nickel chloride); |
| flow rate: | 0.15 ml/min; |
| elution: | A sample is adsorbed in the column with 47.5 ml of A solution, washed with 40 ml of B solution, and eluted with 32 ml of a linear concentration gradient of from B solution 100% to C solution 100%. |

The elution of protein was monitored by measuring absorbance at 280 nm, and the results are shown in Fig. 6. The fractions represented by a hatched bar in Fig. 6 were those fractions which showed Rat2 cell growth inhibiting activity. Effluents were collected by a fraction collector and each fraction was dialyzed against the above described A solution using the above mentioned seamless cellulose membrane and then Rat2 cell growth inhibiting activity was measured by the method as described in 5 of Example 1. The results are shown in Fig. 7.

The fraction IV in Figs. 6 and 7 which showed the growth inhibiting activity was membrane-concentrated with Centriprep-10 (Amicon) and further subjected to chromatography using a dye affinity carrier Red Sepharose CL-6B (Pharmacia)-packed column under the following conditions:

Condition 3:

| | |
|---|---|
| column: | Red Sepharose CL-6B-packed Column (5x50 mm); |
| eluent: | A solution (20 mM phosphate buffer, pH 7.5, 0.1 mM nickel chloride) 3 ml; |
| | B solution (20 mM phosphate buffer, pH 7.5, 1.0 M sodium chloride, 0.1 mM nickel chloride) 3 ml; |
| flow rate: | 0.03 ml/min; |
| elution: | linear concentration gradient from A solution to B solution. |

The elution of protein was monitored by measuring absorbance at 280 nm. Effluents were collected by a fraction collector and Rat2 cell growth inhibiting activity of each fraction was assayed by the method as described in 5 of Example 1. The results are shown in Fig. 8. The gradient elution was commenced at the point of 3.9 ml in the abscissa. The hatched box represents the main fraction of Rat2 cell growth inhibiting activity. The fraction represented by the hatched bar in Fig. 8 was taken as a purified standard.

About 50 μg of Rat2 cell growth inhibiting active substance was obtained by the above mentioned purification methods. The purified standard was analyzed by SDS-polyacrylamide gel electrophoresis under reducing conditions with mercaptoethanol and three bands of molecular weights 39, 40.5 and 41 kilo daltons were detected by silver staining.

The structure of the purified standard was determined by the following method:

Each of the three bands on the SDS-polyacrylamide gel electrophoresis was cut and digested with Lysyl endopeptidase (Wako Pure Chemicals) at 37°C for 8 hours in the presence of 4 M urea in 0.1 M Tris HCl buffer, pH 9.0. The peptide fragments obtained by the enzyme digestion were separated by reverse phase column chromatography and the amino acid sequence was determined by Protein Sequencer PPSQ-10 (Shimadzu Seisakusho). The identical amino acid sequence was obtained from all three bands. The sequence is shown in SEQ ID NOs: 1 and 2. The determined amino acid sequence was searched in the protein database and, as a result, the peptides of SEQ ID NOs: 1 and 2 coincided with the amino acid sequences corresponding to 42-61 residues and 173-191 residues from the N terminal of rat arginase, respectively: J. Biol. Chem., 263, 2245 (1988); Proc. Natl. Acad. Sci. U.S.A., 84, 412 (1987); J. Biol. Chem., 262, 6280 (1987). From the above results, the purified standard was identified as rat arginase.

7. Isolation of Ito cells and assay of Ito cell transformation inhibiting activity

Ito cells were isolated according to the method used by Kawada et al. (Eur. J. Biochem., 213, 815 (1993)).

The isolated Ito cells were adjusted to a cell concentration of 1.7 x $10^5$ per well with Dulbecco MEM medium containing 10% of bovine serum (Virology, 8, 396 (1959)), and seeded in a 12-well cell culture plate (Costar) at 2 ml/well. After the cells were cultured in the presence of 5% carbon dioxide at 37°C for 2 days, the culture medium was exchanged and a medium, to which the liver perfusates LP6-10 prepared in 1 of Example 1 were added at a final concentration of 5% or rat arginase purified in 6 of Example 1 was added at a final concentration of 1 μg/ml, was added at 2 ml/well. The cells were further cultured in the presence of 5% carbon dioxide at 37°C for 4 days. Again the medium was exchanged by the same method as on the second day after the start of cultivation as described above, and the cultivation was continued for additional 7 days. During the cultivation period, the morphological change of Ito cells was observed under an optical microscope.

Fig. 9 shows the change of morphology of Ito cells on 2nd, 6th and 13th day after the start of cultivation. It was observed that Ito cells cultured in Dulbecco MEM medium containing 10% bovine serum transformed into fibroblast-like cells in course of the culture period. On the other hand, when the liver perfusates at a final concentration of 5% and the purified arginase at a final concentration of 1 μg/ml were added, the morphology of Ito cells having fatty granules in the cell was retained and no transformation was observed. Thus, these additives inhibited the transformation of Ito cells into fibroblasts.

8. Assay of Ito cell transformation inhibiting activity of arginases

The Ito cell transformation inhibiting activity of arginases was confirmed by the following method. The rat arginase obtained in 6 of Example 1 and bovine liver arginase (Sigma) were added to the culture solution of Ito cells at a concentration of about 1 μg/ml and 80 μg/ml, respectively, as in 7 of Example 1. As a result, the transformation of Ito cells into fibroblasts was not observed even on 12th day after the addition of sample.

9. Preparation of arginase from a living sample

Forty-eight (48) male SD rats of 250 to 270 g in body weight were provided as in 1 of Example 1. Under anesthsia with diethyl ether, the abdomen was cut, a catheter was inserted into the portal vein, and 20 ml of Ringer solution at 20°C was injected through the portal vein. At that time, the inferior venae cava above the liver was cut so that Ringer solution was removed out. Thus, the liver was made bloodless. Then, tissues around the blood vessels linking to the liver were exfoliated, only the portal vein and inferior venae cava were remained while all the remainder was closed, and then the liver was removed. The liver was subjected to acetone precipitation, heat treatment, and ethanol precipitation according to the method of Shimke et al.: J. Biol. Chem., 239, 3808 (1964). The resultant liver extract was filtered through MILLEX GV (Millipore) and subjected to Red Sepharose CL-6B column chromatography under the following condition A:

Condition A:

column: Red Sepharose CL-6B packed column (25x200 mm);

eluent: A solution (20 mM phosphate buffer, pH 7.5, 0.1 mM manganese chloride) 250 ml;

B solution (20 mM phosphate buffer, pH 7.5, 1.0 M sodium chloride, 0.1 mM manganese chloride) 250 ml;

flow rate: 1.67 ml/min;
elution: linear concentration gradient from A solution to B solution.

The elution of protein was monitored by measuring absorbance at 280 nm. Effluents were collected by a fraction collector and each fraction was estimated according to the method of measuring arginase activity as reported by Garganta and Bond: Analytical Biochemistry, 154, 388 (1986).

Fractions which showed arginase activity were further subjected to chromatography using a cation exchange carrier-packed column under the following condition B:

Condition B:

column: HiLoad SP-Sepharose HP (16/10) (16x100 mm, Pharmacia);
eluent: A solution (10 mM phosphate buffer, pH 7.0);
B solution (10 mM phosphate buffer, pH 7.0, 1.0 M sodium chloride);
flow rate: 3 ml/min;
elution: linear concentration gradient of B solution 0% to 30%, 360 ml.

The elution of protein was monitored by measuring absorbance at 280 nm. Effluents were collected by a fraction collector and the arginase activity of each fraction was assayed by the method as described above. The above purification yielded about 30 mg of arginase.

10. Assay of liver fibrosis inhibiting activity of rat arginase purified from living sample in animal model of hepatocirrhosis

(1) Preparation of animal model of hepatocirrhosis

A hepatocirrhosis inducer used was carbon tetrachloride (Kanto Kagaku-Merck, atomic absorption spectrometric grade, purity 99.9%, Lot. No. 605H1013) which had been diluted with olive oil (Nikko Seiyaku, Japanese Pharmacopoeia Lot No. 244257) at 70% (v/v). The carbon tetrachloride was sterilized with 0.45 μm filter membrane (Costar, μSTAR, Catalogue No. 8112), and the olive oil was sealed in a Medium bottle and both of them were subjected to high pressure steam sterilization (121°C, 1.2 atoms, 20 minutes). Also, the instruments used upon preparation of the below mentioned pharmaceuticals were all subjected to high pressure steam sterilization. Upon dilution, each reagent was taken by a 20 ml glass syringe with a Luer lock (Natsume Seisakusho), two syringes containing the reagent were connected with a connecting needle, and pumping was repeated 50 times to yield a homogeneous solution. The inducer was prepared once a week, sealed in a brown vial bottle and stored in a cool and dark place.

Male SD rats (Nippon SLC, body weight of 220 to 240 g) were purchased, bred about 1 week and divided into a group of 11 animals to which PBS was administered, and groups to which the purified rat arginase from a living sample obtained by the method described in 9 of Example 1 was administerd at 60 μg/animal/day and 200 μg/animal/day, each of 13 animals.

After inhalation anesthesia with diethyl ether, 1 ml of carbon tetrachloride preparation (0.7 ml carbon tetrachloride per kg body weight) was intramuscularly adminidtered to the femur under anesthesia by means of a 1 ml plastic syringe with a Luer lock (Natsume Seisakusho). The administration was done twice a week, on Thuesday and Friday every week, for 6 weeks.

(2) Methods of administration of liver perfusate and Ringer solution

After the rats were treated by diethyl ether inhalation anesthesia prior to the administration (or after the administration on Thursday and Friday when carbon tetrachloride was to be administered), 0.5 ml of PBS or 2.5 ml of PBS containing 120 μg/ml or 200 μg/ml of the living sample derived purified rat arginase was administered into the penis veniplex by means of a disposable plastic syringe (Terumo). The administration was done every day on 3rd to 6th weeks after the start of administration of carbon tetrachloride.

(3) Necrotomy and treatment

On 3rd day after the last administration of carbon tetrachloride, the rats were sacrificed and the liver was removed out by dissection of the rats. Specimens of 2 to 3 mm in thickness from each lobe of the liver were fixed with formalin. One week after the fixation, the pathological tissue specimens of the liver were stained with Hematoxylin Eosin (HE) and Fast green, Sirius red (FS) staining methods to specifically stain both collagenous and elastic fibers. The stages of fibrosis were classified into following 4 grades:

Grade 0: No production of new collagen is observed;
Grade 1: The production of collagen starts;
Grade 2: The production of collagen is completed in the portal regions; and
Grade 3: Hepatocirrhosis occurs.

The liver fibrosis grade of each individual was judged according to the typical fiber stained image in each grade shown in Fig. 2 as liver fibrosis grade standards.

The liver fibrosis grade of the PBS administered group and the living sample drived purified rat arginase (60 µg/animal/day and 200 µg/animal/day) administered groups are shown in Table 3. In the living sample derived purified rat arginase (200 µg/animal/day) administered group, the hepatoirrhosis is inhibited.

Table 3

| Group tested | Incidence (%) | | | | |
|---|---|---|---|---|---|
| | number of animals | Grade | | | |
| | | 0 | 1 | 2 | 3 |
| PBS administered | 11 | 0 | 0 | 27.3 | 72.7 |
| Living sample derived rat arginase administered (60 µg/animal/day) | 13 | 0 | 0 | 30.7 | 69.3 |
| Living sample derived rat arginase administered (200 µg/animal/day) | 13 | 0 | 7.7 | 38.5 | 53.8 |

Example 2

To prepare an arginine-degrading enzyme having the fibrosis inhibiting activity in a large scale, the arginine-degrading enzyme was prepared by gene recombinant method as described below.

1. Construction of expression vector pNS (Fig. 10)

To construct an expression vector in which SalI site had been inserted between NdeI site and terminator of pET-11a purchased from Novagen, the BamHI site of pET-11a was cut to form a blunt end and a synthetic linker containing a SalI site was inserted between the blunt end and the NdeI site.

Two (2) µg of pET-11a (Novagen) was dissolved in 20 µl of a buffer comprising 20 mM Tris-HCl (pH 8.5), 10 mM $MgCl_2$, 100mM KCl, 1mM dithiothreitol (hereinafter abbreviated as DTT), hereinafter abbreviated as Buffer K), and 10 units of BamHI (Takara Shuzo, hereinafter restriction enzymes used were manufactured by Takara Shuzo unless otherwise specified) was added to carry out digestion reaction at 37°C for 2 hours. After ethanol precipitation, the precipitate was dissolved in 30 µl of DNA polymerase I buffer [50 mM Tris-HCl (pH 7.5), 10 mM $MgCl_2$, 0.1 mM dATP (deoxyadenosine triphosphate), 0.1 mM dCTP (deoxycytidine triphosphate), 0.1 mM dGTP (deoxyguanosine triphosphate), 0.1 mM TTP (thymidine triphosphate)] and 6 units of Escherichia coli DNA polymerase I Klenow fragment (Takara Shuzo) was added to react at 37°C for 60 minutes. Thus, the 5' cohesive end generated by the BamHI digestion was converted into a blunt end. The reaction mixture was subjected to agarose gel electrophoresis and a DNA fragment of about 5.7 kb was recovered.

The recovered DNA fragment was dissolved in 20 µl of a buffer comprising 50 mM Tris-HCl (pH 7.5), 10 mM $MgCl_2$, 100 mM NaCl, and 1 mM DTT, hereinafter abbreviated as Buffer H, 10 units of NdeI was added to carry out digestion reaction at 37°C for 2 hours. After the reaction solution was subjected to agarose gel electrophoresis [40 mM tris-acetate buffer (pH 8.0) containing 0.7% ararose, TAE buffer (1 mM EDTA); TAE buffer was used in all agarose gel electrophoresis], a DNA fragment of about 5.7 kb was recovered.

Separately, to ligate the NdeI site with the BamHI site which was converted into a blunt end, DNA linkers of SEQ ID NOs. 3 and 4 [designated as SNS-01 (29mer) and SNS-02 (31mer)] were synthesized using a DNA synthesizer (Applied Biosystems, 380A).

The pET-11a derived BamHI-NdeI fragment (5.7 kb, 0.2 μg) obtained above was dissolved in 9 μl of a solution comprising 50 mM Tris-HCl (pH 7.8), 10 mM $MgCl_2$, 10 mM DTT, 1 mM adenosine triphosphate (hereinafter abbreviated as ATP) and 25 μg/ml bovine serum albumin (hereinafter abbreviated as BSA), hereinafter abbreviated as T4 ligase buffer, and 0.01 μg of the above mentioned DNA linkers and 2,000 units of T4 DNA ligase (New England Biolab) were added to carry out ligation reaction at 16°C for 16 hours.

The reaction solution was used to transform Escherichia coli strain HB101 (Gene, 2, 75 (1977)) by the method of Cohen et al. (Proc. Natl. Acad. Sci. U.S.A., 69, 2110 (1972)) [this method was herein used to transform Escherichia coli] to yield an ampicillin resistant clone. A plasmid was isolated from this transformant according to the known method (Nucleic Acids Res., 7, 1513 (1979)) [this method was herein used to isolate plasmids unless otherwise indicated]. The structure of the obtained plasmid was confirmed by restriction enzyme digestion. This plasmid is designated as pNS.

A plasmid was prepared from the above mentioned transformant by using a 〉plasmid 〈 midi kit (Qiagen, Product No. 12143). The obtained plasmid was ethanol precipitated and then dissolved in a buffer comprising 10 mM Tris-HCl (pH 8.0), 1 mM EDTA (sodium ethylenediamine tetraacetate), hereinafter abbreviated as TE Buffer.

2. Production of human arginase

(1) Cloning of arginase cDNA from human liver and construction of human arginase expression plasmid (Fig. 11)

Using total RNA extracted from a human liver tissue as a template and oligo dT as a primer, a single stranded cDNA was synthesized. This single stranded cDNA was used as a template to prepare the gene portion coding for the full length human arginase by polymerase chain reaction, hereinafter abbreviated as PCR, and incorporated into the secretory expression vector constructed in 1 of Example 2.

From 5 μg of total RNA extracted from human liver tissue according to the conventional method (Biochemistry, 18, 5294 (1977)), a single stranded cDNA was synthesized using a single stranded cDNA synthesizer kit SuperScript™ (GIBCO BRL) and oligo dT as a primer. The thus obtained single stranded cDNA (0.2%) was used as a template for PCR.

As the PCR primers, two synthetic DNAs of SEQ ID NOs. 5 and 6 [designated as SSA-03 (44mer) and SSA-04 (36mer)] were synthesized using Applied Biosystems 380A DNA synthesizer.

Since SSA-03 and SSA-04 are designed in such a manner that NdeI and SalI sites can be introduced, respectively, DNA fragments amplified by PCR can be incorporated between the NdeI and SalI sites of pNS after digested with NdeI and SalI. PCR was performed by using GeneAmp™ DNA Amplification Reagent Kit with AmpliTaq™ Recombinant Taq DNA Polymerase (Takara Shuzo). The reaction solution was prepared according to the method described in the specification attached to the kit and 30 cycles of incubation at 94°C for 30 seconds, 65°C for 1 minute and 72°C for 2 minutes were carried out using PERKIN ELMER CETUS DNA Thermal Cycler (Takara Shuzo) followed by further incubation at 72°C for 7 minutes. After the reaction was completed, chloroform extraction and ethanol precipitation were carried out.

The precipitate resulting from the above mentioned ethanol precipitation was dissolved in 30 μl of Buffer H and 20 units of NdeI and 20 units of SalI were added to perform digestion reaction at 37°C for 2 hours. The reaction solution was subjected to 1% agarose gel electrophoresis and DNA fragment of about 1.0 kb was recovered.

Two (2) μg of pNS was dissolved in 30 μl of Buffer H and 20 units of NdeI and 20 units of MluI were added to perform digestion reaction at 37°C for 2 hours. The reaction solution was subjected to 1% agarose gel electrophoresis and DNA fragment of about 0.8 kb was recovered.

Two (2) μg of pNS was dissolved in 30 μl of Buffer H and 20 units of MluI and 20 units of SalI were added to perform digestion reaction at 37°C for 2 hours. The reaction solution was subjected to 0.7% agarose gel electrophoresis and DNA fragment of about 4.8 kb was recovered.

The NdeI-SalI fragment (1.0 kb, 0.1 μg) derived from DNA amplified by PCR and the NdeI-MluI fragment (0.8 kb, 0.02 μg) and MluI-SalI fragment (4.8 kb, 0.1 μg) derived from pNS were dissolved in 9 μl of T4 ligase buffer. T4 DNA ligase (400 units) was added to perform ligation reaction at 16°C for 16 hours.

The reaction solution was used to transform Escherichia coli strain XL1Blue (StrataGene) by the method of Cohen et al. to yield an ampicillin resistant clone. A plasmid was isolated from the transformant according to the known method and the structure thereof was confirmed by restriction enzyme digestion. This plasmid is designated as pNS-hArg1.

The nucleotide sequence of the pNS-hArg1 obtained above was determined using Taq DyeDeoxy™ Terminator Cycle Sequencing Kit, which is a kit for determining nucleotide sequences (Applied Biosystems). The thus determined nucleotide sequence is shown in SEQ ID NO.: 7. Consequently, it was revealed that the gene cloned in this Example codes for a protein comprising 322 amino acids as shown in SEQ ID NO.: 8 and this amino acid sequence coincides with that of human arginase reported by Takiguchi et al. (Nucleic Acids Res., 16, 8789 (1988)).

(2) Expression of human arginase in Escherichia coli

The plasmid vector pNS-hArg1 constructed in 1 of Example 2 was used to transform Escherichia coli strain BL21 by the method of Cohen et al. to yield an ampicillin resistant strain Escherichia coli BL21/pNS-hArg. Into a 300 ml Erlenmeyer flask equipped with a baffle, 20 ml of L-broth [10 g bactotryptone, 5 g yeast extract (both of DIFCO), 5 g NaCl/1 L $H_2O$] was placed and subjected to high pressure steam sterilization (121°C, 1.2 atom, 20 minutes). A filter sterilized ampicillin solution was added at a final concentration of 100 μg/ml and the transformant ccoli BL21/pNS-hArg was seeded and shaking cultured (220 rpm) at 37°C for 8 hours. To each of the three 1 L Erlenmeyer flasks equipped with a baffle, 250 ml of L-broth was added and subjected to high pressure steam sterilization (121°C, 1.2 atom, 20 minutes). A filter sterilized ampicillin solution was added at a final concentration of 100 μg/ml. To each flask, 2.5 ml of the above mentioned 20 ml culture was added and shaking cultured (220 rpm) at 37°C for 15 hours. Further, to a 30 L jar fermentor (Maruhishi Bioengi, MSJ-N2 Model 30L), 15 L of M9 medium [1.514% $Na_2HPO_4.12H_2O$, 0.3% $KH_2PO_4$, 0.5% NaCl, 0.1% $NH_4Cl$, 0.2% peptone (Kyokuto Seiyaku Kabushiki Kaisha), 0.246 g/L $MgSO_4$, 8 mg/L vitamin B1, 990 μg/L $FeSO_4$, 880 μg/L $ZnSO_4$, 393 μg/L $CuSO_4$, 72 μg/L $MnSO_4$, 88 μg/L $Na_2B_4O_4$, 37 μg/L $(NH_4)_6Mo_7O_{24}$] containing 0.5% glycerol was placed and subjected to high pressure steam sterilization (121°C, 1.2 atom, 20 minutes). A filter sterilized ampicillin solution was then added at a final concentration of 100 μg/ml. The above mentioned three bottles of 250 ml cultures were added. After culturing with under aerobic conditions (450 rpm, 1 vvm) at 37°C, at the point that OD600 reached 0.8, an expression inducer isopropyl-β-D-thiogalactopyranoside (hereinafter abbreviated as IPTG) was added at a final concentration of 0.1 mM to induce the expression of human arginase. Four hours after the start of the induction of expression, the cultivation was stopped and the culture was centrifuged to yield 168 g (wet weight) of cells.

(3) Purification of human arginase derived from Escherichia coli

The human arginase from the cultured cells obtained in 2 (2) of Example 2 above was purified in the following manner:

The following procedures for purification of human arginase were all carried out at 0 to 4°C. The cultured cells (wet weight 168 g) were suspended in 600 ml of PBS and the Escherichia coli cells were disrupted once at 200 kg/cm$^2$ and four times at 500 kg/cm$^2$ using Manton Gaulin Homogeniger (Doei Shoji). (This procedure was also done under cooling with ice: since the suspension temperature raised upon disruption, the cell suspension was cooled below 4°C prior to the next disruption.) The thus disrupted cell suspension was centrifuged at 10,000 rpm for 40 minutes. To the supernatant, an equal amount of PBS containing 80% saturated ammonium sulfate was added and allowed to stand for 3 hours to effect ammonium sulfate precipitation. After further centrifugation at 10,000 rpm for 40 minutes, the supernatant was purified by hydrophobic interaction chromatography. The hydrophobic interaction chromatography was performed under the following condition i:

Condition i:

| | |
|---|---|
| column: | Phenyl-Sepharose Fast Flow (low substitution, Pharmacia, 50x240 mm); |
| eluent: | A solution (PBS, pH 7.2, 40% saturated ammonium sulfate) 1 L; |
| | B solution (PBS, pH 7.2) 1 L; |
| flow rate: | 470 ml/hr; |
| elution: | linear concentration gradient from A solution to B solution. |

The effluents were fractionated into 45 ml fractions and each fraction was analyzed by SDS-polyacrylamide gel electrophoresis under reducing conditions with mercaptoethanol. The fractions containing bands of human arginase around 38 kilo daltons in molecular weight were collected, which was designated as Phenyl-Sepharose human arginase fraction.

Using a seamless cellulose membrane (Viskase Sales Corp.), 1750 ml of the Phenyl-Sepharose human arginase fraction was dialyzed against 60 L of 10 mM phosphate buffer (pH 7.0) twice (12 hours, 3 hours). The dialysate was purified by cation exchange chromatography. The cation exchange chromatography was carried out under the following conditions. In the cation exchange chromatography, pyrogen free water made by reverse osmotic purified water system (Dicel Chemical Industries, Ltd., MOLSEP, SYSTEM BX-SU) was used.

Condition ii:

| | |
|---|---|
| column: | SP-Sepharose Fast Flow (Pharmacia, 50x200 mm); |
| eluent: | A solution (10 mM phosphate buffer, pH 7.0) 1 L; |
| | B solution (10 mM phosphate buffer, pH 7.0, 0.5 M sodium chloride) 1 L; |
| flow rate: | 400 ml/hr; |
| elution: | linear concentration gradient from A solution to B solution. |

The effluents were fractionated into 42 ml fractions and each fraction was analyzed by SDS-polyacrylamide gel electrophoresis under reducing conditions with mercaptoethanol. The fractions containing bands of human arginase around 38 kilo daltons in molecular weight were collected, which was designated as SP-Sepharose human arginase fraction.

The SP-Sepharose human arginase fraction contained 1360 mg of proteins and the purity of human arginase was 90% or higher. Further, the arginase activity was determined by the arginase activity assay as reported by Garganta and Bond: Analytical Biochemistry, 154, 388-394 (1986) and was found to be 1990 U/ml. The amount of proteins herein means the value calculated as bovine serum albumin using BIO-RAD protein assay kit.

Furthermore, the rat fibroblast Rat-2 growth inhibiting activity was assayed by the method shown in 6 of Example 1; at 1.5 μg/ml of human arginase, a growth inhibiting activity identical with that when 1.25% of LP6 to LP10 were added was observed.

3. Production of rat arginase

(1) cloning of arginase cDNA from a rat liver and construction of rat arginase expression plasmid (Fig. 12)

PolyA RNA was extracted from rat liver tissue and a single stranded cDNA was synthesized using oligo dT as a primer. This single stranded cDNA was used as a template to prepare the gene portion coding for the full length rat arginase by polymerase chain reaction (PCR), and incorporated into the secretory expression vector pNS constructed in 1 of Example 2.

From 1 g of the rat liver tissue, about 50 μg of polyA RNA was obtained using a polyA RNA extraction kit Fast Track (In Vitro Gen). Actual reagents and method used were according to the protocol attached to the kit.

From 1 μg of 50 μg of polyA RNA obtained above, a single stranded cDNA was synthesized using a single stranded cDNA synthesizer kit SuperScript™ (GIBCO BRL) and oligo dT as a primer. Ten (10) percent of the single stranded cDNA obtained above was used as a template for PCR.

As the PCR primers, synthetic DNAs of SEQ ID NOs. 11 and 12 [SSA-07 (44mer) and SSA-08 (36mer)] were synthesized using Applied Biosystems 380A DNA synthesizer.

Since SSA-07 and SSA-08 are designed in such a manner that NdeI and SalI sites can be introduced, respectively, DNA fragments amplified by PCR can be incorporated between the NdeI and SalI sites of pNS after digested with NdeI and SalI. PCR was performed by using GeneAmp™ DNA Amplification Reagent Kit with AmpliTaq™ Recombinant Taq DNA Polymerase (Takara Shuzo). The reaction solution were prepared according to the method described in the specification attached to the kit and 30 cycles of incubation at 94°C for 30 seconds, 65°C for 1 minute and 72°C for 2 minutes were carried out using PERKIN ELMER CETUS DNA Thermal Cycler (Takara Shuzo) followed by incubation at 72°C for additional 7 minutes. After the reaction was completed, chloroform extraction and ethanol precipitation were carried out.

The precipitate resulting from the above mentioned ethanol precipitation was dissolved in 30 μl of Buffer H and 20 units of NdeI and 20 units of SalI were added to perform digestion reaction at 37°C for 2 hours. The reaction solution was subjected to 1% agarose gel electrophoresis and DNA fragment of about 1.0 kb was recovered.

Two (2) μg of pNS was dissolved in 30 μl of Buffer H and 20 units of NdeI and 20 units of MluI were added to perform digestion reaction at 37°C for 2 hours. The reaction solution was subjected to 1% agarose gel electrophoresis and DNA fragment of about 0.8 kb was recovered.

Two (2) μg of pNS was dissolved in 30 μl of Buffer H and 20 units of MluI and 20 units of SalI were added to perform digestion reaction at 37°C for 2 hours. The reaction solution was subjected to 0.7% agarose gel electrophoresis and DNA fragment of about 4.8 kb was recovered.

The NdeI-SalI fragment (1.0 kb, 0.1 μg) derived from DNA amplified by PCR and the NdeI-MluI fragment (0.8 kb, 0.02 μg) and MluI-SalI fragment (4.8 kb, 0.1 μg) derived from pNS were dissolved in 9 μl of T4 ligase buffer. T4 DNA ligase (400 units) were added to perform ligation reaction at 16°C for 16 hours.

The reaction solution was used to transform Escherichia coli strain XL1Blue (StrataGene) by the method of Cohen et al.to yield an ampicillin resistant clone. A plasmid was isolated from the transformant according to the known method and the structure thereof was confirmed by restriction enzyme digestion. This plasmid is designated as pNS-rArg1.

The nucleotide sequence of the pNS-rArg1 obtained above was determined using Taq DyeDeoxy™ Terminator Cycle Sequencing Kit, which is a kit for determining nucleotide sequences (Applied Biosystems). The thus determined nucleotide sequence is shown in SEQ ID NO.: 9. Consequently, it was revealed that the gene cloned in this Example codes for a protein comprising 323 amino acids as shown in SEQ ID NO.: 10 and this amino acid sequence coincides with that of rat arginase reported by Ohtake et al. (J. Biol. Chem., 263, 2245 (1988)).

(2) Expression of rat arginase in Escherichia coli

The plasmid vector pNS-rArg1 constructed in 3 (1) of Example 2 was used to transform Escherichia coli strain BL21 by the method of Cohen et al. to yield an ampicillin resistant strain Escherichia coli BL21/pNS-rArg1. To a 300 ml Erlenmeyer flask equipped with a baffle, 20 ml of L-broth was placed and subjected to high pressure steam sterilization (121°C, 1.2 atom, 20 minutes). A filter sterilized ampicillin solution was added at a final concentration of 100 μg/ml and the transformant Escherichia coli BL21/pNS-hArg1 was seeded and shaking cultured (220 rpm) at 37°C for 8 hours. To the 1 L Erlenmeyer flask equipped with a baffle, 125 ml of L-broth was added and subjected to high pressure steam sterilization (121°C, 1.2 atom, 20 minutes). A filter sterilized ampicillin solution was added at a final concentration of 100 μg/ml. To the flask, 1.25 ml of the above mentioned 20 ml culture was added and shaking cultured (220 rpm) at 37°C for 15 hours. Further, to a 5 L jar fermentor (Mitsuwa Biosystem, KMJ-5C-6U), 2.5 L of M9 medium containing 0.5% glycerol was placed and subjected to high pressure steam sterilization (121°C, 1.2 atom, 20 minutes). A filter sterilized ampicillin solution was then added at a final concentration of 100 μg/ml and 125 ml of the above mentioned culture solution was added. After culturing with under aerobic conditions (600 rpm, 1 vvm) at 37°C. At the point that OD600 reached 0.8, an expression inducer IPTG was added at a final concentration of 0.1 mM to induce the expression of human arginase. Four hours after the start of the induction of expression, the cultivation was stopped and the culture solution was centrifuged to yield 20 g (wet weight) of cells.

(3) Purification of rat arginase derived from Escherichia coli

The rat arginase from the cultured cells obtained in 3 (2) of Example 2 above was purified in the following manner:

The following procedures for purification of the rat arginase were all carried out at 0 to 4°C. From the resultant cultured cells (wet weight 20 g), 8 g was weighed and suspended in 40 ml of PBS. The Escherichia coli cells were disrupted three times in a ultrasonic disruptor (BRANSON, SONIFIER, CELL DISRUPTOR 200, flat tip, output level 10) for 10 seconds. (This procedure was also done under cooling with ice: since the suspension temperature raised upon disruption, the cell suspension was cooled below 4°C prior to the next disruption.) The thus disrupted cell suspension was centrifuged at 15,000 rpm for 20 minutes. To the supernatant, an equal amount of PBS containing 80% saturated ammonium sulfate was added and allowed to stand for 3 hours to effect ammonium sulfate precipitation. After further centrifugation at 15,000 rpm for 20 minutes, the supernatant was purified by hydrophobic interaction chromatography. The hydrophobic interaction chromatography was performed under the following condition iii:

Condition iii:

| | |
|---|---|
| column: | Phenyl-Sepharose Fast Flow (low substitution, Pharmacia, 10x130 mm); |
| eluent: | A solution (PBS, pH 7.2, 40% saturated ammonium sulfate) 25 ml; |
| | B solution (PBS, pH 7.2) 25 ml; |
| flow rate: | 10 ml/hr; |
| elution: | linear concentration gradient from solution A to B solution. |

The effluents were fractionated into 5 ml fractions and each fraction was analyzed by SDS-polyacrylamide gel electrophoresis under reducing conditions with mercaptoethanol. The fractions containing bands of rat arginase around 38 kilo daltons in molecular weight were collected, which was designated as Phenyl-Sepharose rat arginase fraction.

Using a seamless cellulose membrane (Viskase Sales Corp.), 20 ml of the Phenyl-Sepharose rat arginase fraction was dialyzed against 2.5 L of 10 mM phosphate buffer (pH 7.0) twice (12 hours, 3 hours). The dialysate was purified by cation exchange chromatography. The cation exchange chromatography was carried out under the following condition iv. In the cation exchange chromatography, pyrogen free water made by reverse osmotic purified water system (Dicel Chemical Industries, Ltd., MOLSEP, SYSTEM BX-SU) was used.

Condition iv:

| | |
|---|---|
| column: | SP-Sepharose Fast Flow (Pharmacia, 25x100 mm); |
| eluent: | A solution (10 mM phosphate buffer, pH 7.0) 150 ml; |
| | B solution (10 mM phosphate buffer, pH 7.0, 0.5 M sodium chloride) 150 ml; |
| flow rate: | 50 ml/hr; |
| elution: | linear concentration gradient from A solution to B solution. |

The effluents were fractionated into 10 ml fractions and each fraction was analyzed by SDS-polyacrylamide gel electrophoresis under reducing conditions with mercaptoethanol. The fractions containing bands of rat arginase around 38 kilo daltons in molecular weight were collected, which was designated as SP-Sepharose rat arginase fraction.

The SP-Sepharose rat arginase fraction contained 23 mg of proteins and the purity of rat arginase was 90% or higher. Further, the arginase activity was assayed by a method similar to that in 2 (3) of Example 2 and was found to be 1460 U/ml. The amount of proteins herein means the value calculated as bovine serum albumin using BIO-RAD protein assay kit.

Furthermore, the rat fibroblast Rat-2 growth inhibiting activity was assayed by the method shown in 6 of Example 1; at 1.5 μg/ml of the rat arginase, a growth inhibiting activity identical with that when 1.25% of LP6 to LP10 were added was observed.

Example 3

1. Injections

In a conventional procedure, an injection having the following composition was prepared:

Twenty-five (25) mg of the human arginase obtained in 2 of Example 2 was dissolved in 80 ml of phosphate buffered saline (PBS) solution and 2 mg of polysorbate 80 (Wako Pure Chemical), 100 mg of human serum albumin (Sigma), 1.26 mg of $MnCl_2$ and 1.5 g of D-mannitol were added. The volume was adjusted to 100 ml with PBS. The resulting solution was aseptically filtered through a 0.22 μm Disposable membrane filter and aseptically filled into glass vials in an amount of 2 ml per glass vial to make an injectable preparation containing 0.5 mg of the active ingredient in a vial.

| | |
|---|---|
| Arginase | 0.5 mg |
| Polysorbate 80 | 0.04 mg |
| Human serum albumin | 2.0 mg |
| D-mannitol | 30 mg |
| $MnCl_2$ | 0.0252 mg |
| NaCl | 16 mg |
| KCl | 0.4 mg |
| $KH_2PO_4$ | 0.4 mg |
| $Na_2HPO_4.12H_2O$ | 5.8 mg |
| Total volume | 2.0 ml |

2. Injections

In a conventional procedure, an injection having the following composition was prepared:

Twenty-five (25) mg of the human arginase obtained in 2 of Example 2 was dissolved in 80 ml of PBS solution and 2 mg of polysorbate 80 (Wako Pure Chemical) and 1.5 g of D-mannitol were added. The volume was adjusted to 100 ml with PBS. The resulting solution was aseptically filtered through a 0.22 μm Disposable membrane filter and aseptically filled into glass vials in an amount of 2 ml per glass vial to make an injectable preparation containing 0.5 mg of active ingredient in a vial.

| | |
|---|---|
| Arginase | 0.5 mg |
| Polysorbate 80 | 0.04 mg |
| D-mannitol | 30 mg |
| NaCl | 16 mg |
| KCl | 0.4 mg |
| $KH_2PO_4$ | 0.4 mg |
| $Na_2HPO_4.12H_2O$ | 5.8 mg |
| Total volume | 2.0 ml |

3. Injections

In a conventional procedure, an injection having the following composition was prepared:
Twenty-five (25) mg of the human arginase obtained in 2 of Example 2 was dissolved in 80 ml of PBS solution and 1.26 mg of $MnCl_2$ was added. The volume was adjusted to 100 ml with PBS. The resulting solution was aseptically filtered through a 0.22 μm Disposable membrane filter and aseptically filled into glass vials in an amount of 2 ml per glass vial to make an injectable preparation containing 0.5 mg of active ingredient in a vial.

| | |
|---|---|
| Arginase | 0.5 mg |
| $MnCl_2$ | 0.0252 mg |
| NaCl | 16 mg |
| KCl | 0.4 mg |
| $KH_2PO_4$ | 0.4 mg |
| $Na_2HPO_4.12H_2O$ | 5.8 mg |
| Total volume | 2.0 ml |

4. Injections

In a conventional procedure, an injection having the following composition was prepared:
Twenty-five (25) mg of the human arginase obtained in 2 of Example 2 was dissolved in 80 ml of PBS solution and the volume was adjusted to 100 ml with PBS. The resulting solution was aseptically filtered through a 0.22 μm Disposable membrane filter and aseptically filled into glass vials in an amount of 2 ml per glass vial to make an injectable preparation containing 0.5 mg of active ingredient in a vial.

| | |
|---|---|
| Arginase | 0.5 mg |
| NaCl | 16 mg |
| KCl | 0.4 mg |
| $KH_2PO_4$ | 0.4 mg |

(continued)

| | |
|---|---|
| $Na_2HPO_4.12H_2O$ | 5.8 mg |
| Total volume | 2.0 ml |

Example 4

1. Production of polyethylene glycol (hereinafter abbreviated as PEG) modified rat arginase

In the follownig tests, the pyrogen free water described in 2 (3) of Example 2 was used.

The rat arginase (707 mg) prepared according to the method of 3 of Example 2 was filter sterilized through a cellulose acetate membrane (SARTORIUS, Sartolab P20 plus) and subjected to cation exchange chromatography under the following condition α to reduce the level of pyrogen in the protein solution.

Condition α:

| | |
|---|---|
| column: | HiLoad SP Sepharose HP (Pharmacia, 26x100 mm); |
| eluent A: | 10 mM phosphate buffer, pH 7.5, 125 ml; |
| eluent B: | 10 mM phosphate buffer, pH 7.5, 0.25 M sodium chloride, 125 ml; |
| flow rate: | 468 ml/hr; |
| elution: | linear concentration gradient from eluent A to eluent B; |
| temperature: | 4°C. |

The elution of the rat arginase from HiLoad SP Sepharose HP column was monitered by measuring absorption at 280 nm.

The resulting protein solution containing 515 mg of the thus obtained rat arginase was prepared so as to be 107.5 ml of 50 mM phosphate buffer (pH 7.3) containing a final concentration of 0.1 mM of manganese chloride. To this solution, 4.55 g of methoxy polyethylene glycol propionic acid N-hydroxysuccinimide ester (SHEARWATER POLYMERS, United States, average molecular weight about 5,000) dissolved in 18 ml of dimethylsuslfoxide was added and stirred at 4°C for 1 hour and 30 minutes. Further, 3.2 ml of 1 M ethanolamine was added to the reaction mixture to stop the reaction.

The reaction solution was concentrated to 50 ml with a ultrafilter membrane (Centriprep-10, Millipore) and subjected to gel filtration chromatography under the following condition β to remove unreacted PEG modification reagents:

Condition β:

| | |
|---|---|
| column: | Sephacryl S-200 (Pharmacia, 5x42 cm) and Sephacryl S-200 (Pharmacia, 5x87 cm) in series; |
| eluent: | (PBS, pH 7.2, 0.1 mM manganese chloride) 3 L; |
| flow rate: | 252 mL/hr; |
| temperature: | 4°C. |

The elution of the PEG modified rat arginase from Sephacryl S-200 column was monitered by measuring absorption at 280 nm.

The resulting PEG modified rat arginase contained 478 ml of proteins and the purity was 95% or higher. The amount of proteins in the PEG modified rat arginase was determined by the absorption at 280 nm and calculated on the basis of the absorption at 280 nm of the rat arginase standards whose protein concentrations were quantitatively determined by the method described in 3 (3) of Example 2.

A matrix-assited laser disorption ionizing time-of-flight mass spectrometer (Bruker, Reflex) was used to measure the moleular weight of the PEG modified rat arginase and a signal was obtained which indicated the peak molecular weight of 74,000 daltons.

Further, the arginase activity was assayed by the method similar to that in 2 (3) of Example 2 and found to be 2358 U/ml. In the rat fibroblast Rat-2 growth inhibiting activity assay by the method as shown in 6 of Example 1, 1.5 μg/ml of the PEG modified rat arginase showed growth inhibiting activities identical with that obtained when 1.25% LP6 to LP10 were added.

2. Kinetics of PEG modified rat arginase in rat blood

The change with the passage of time in arginase activity and arginine concentration in the blood of rats to which the PEG modified rat arginase was administered was investigated.

(1) Administration method

Male SD rats (body weight of 220 to 240 G) were purchased from Nippon SLC and tested after about 1 week breeding. Samples to be given were the rat arginase obtained in 3 of Example 2, the PEG modified rat arginase obtained in 1 of Example 4, and PBS containing 0.1 mM manganese chloride as a negative control. The rat arginase and PEG modified rat arginase were adjusted to a protein concentration of 200 μg/ml with PBS containing 0.1 mM manganese chloride. After inhalation anesthesia with diethyl ether, each sample was administered to penis veniplex at a dose of 0.5 ml per rat by means of a disposable plastic syringe (Terumo).

(2) Interval and method of bleeding

To determine the change with the passage of time, the blood was taken from the same individual at 1, 3, 6 and 24 hours or 1, 3, 7, 10 and 14 days. The animals were bled from the tail veins at 0.3 ml per animal by a method that causes a minimum effect on the animals. Each rat was fixed in a retaining trap (Natsume Seisakusho) so that it could move only the tail freely, and a disposable injection needle (Terumo, 23x1" ) was cut at the part connecting to the syringe and inserted into the tail vein. The vein blood dripped from the cut end was received in a sample tube (Eppendorph, volume of 1.5 ml) containing 10 μl of 5% EDTA.2K solution as an anticoagulant. After slightly shaking to confirm the absence of coagulation, the blood was centrifuged at 3,000 rpm for 10 minutes to yield plasma. The plasma sample was freezed and stored at -80°C until analysis. Prior to use, the sample was thawed and centrifuged at 3,000 rpm for 10 minutes to remove insoluble matters.

(3) Measurement of arginine concentration in blood

Five μl of the rat plasma obtained in 2 (2) of Example 4 was derivatized with 6-aminoquinolyl-N-hydroxysuccinimidyl carbamate (Nippon Waters) according to Cohen and Michaud method (Anal. Biochem., 211, 279 (1993)) and then subjected to an amino acid analysis.

The results of measurement of arginine concentration in blood of the PBS administered control group, rat arginase administered group and PEG modified rat arginase administered group are shown in Figs. 13 and 14. Sustained inhibiting effects on arginine concentration in the rat bloods were observed by the PEG modified rat arginase administration.

(4) Assay of arginase activity in blood

The arginase activity in the rat plasma obtained in 2 (2) of Example 4 was assayed according to the method of Baston et al. (Anal. Biochem., 191, 384 (1990)): ornithine produced by hydrolysis of the substrate arginine with arginase was quantitatively determined by amino acid analysis and the activity of arginase that produces 1 μmol of ornithine per minute at 37°C was defined as 1 U. The produced ornithine was quantitatively determined by the amino acid analysis as described in 2 (3) of Example 4.

The results of assay of blood arginase activity of the PBS administered group, rat arginase administered group and PEG modified rat arginase administerd group are shown in Figs. 15 and 16. From Figs. 15 and 16, the half life in blood was calculated to be 1 hour and 45 hours in the rat arginase and PEG modified rat arginase, respectively. Thus, the PEG modified arginase had an improved stability in a living body and could sustain the action of arginase for a long time.

Industrial Applicability

The tissue fibrosis inhibiting agents according to the present invention can be used to inhibit the tissue fibrosis in liver, kidney, lung, pancreas, skin, etc. Further, the tissue fibrosis inhibiting agents according to the present invention have excellent stability in blood and organ delivery properties.

SEQUENCE LISTING

SEQ ID NO : 1

SEQUENCE LENGTH : 2 0

SEQUENCE TYPE : amino acid

STRANDEDNESS : single

TOPOLOGY : linear

MOLECULE TYPE : peptide

ORIGINAL SOURCE

ORGANISM : Rattus norvegicus

TISSUE TYPE : liver

SEQUENCE DESCRIPTION:

```
Glu Thr Glu Tyr Asn Val Arg Asp His Gly Asp Leu Ala Phe Val Asp
  1               5                  10                  15
Val Pro Asn Asp
             20
```

SEQ ID NO : 2

SEQUENCE LENGTH : 1 9.

SEQUENCE TYPE : amino acid

STRANDEDNESS : single

TOPOLOGY : linear

MOLECULE TYPE : peptide

ORIGINAL SOURCE

ORGANISM : Rattus norvegicus

TISSUE TYPE : liver

SEQUENCE DESCRIPTION:

```
Asp Ile Val Tyr Ile Gly Leu Arg Asp Val Asp Pro Gly Glu His Tyr
  1               5                  10                  15
Ile Ile Lys
```

SEQ ID NO : 3

SEQUENCE LENGTH : 29

SEQUENCE TYPE : nucleic acid

STRANDEDNESS : single

TOPOLOGY : linear

MOLECULE TYPE : Other nucleic acid synthetic DNA

SEQUENCE DESCRIPTION:

GATAAGCTTG GGCTGCAGGT CGACTTACA 29

SEQ ID NO : 4

SEQUENCE LENGTH : 31

SEQUENCE TYPE : nucleic acid

STRANDEDNESS : single

TOPOLOGY : linear

MOLECULE TYPE : Other nucleic acid synthetic DNA

SEQUENCE DESCRIPTION:

TATGTAAGTC GACCTGCAGC CCAAGCTTAT C 31

SEQ ID NO : 5

SEQUENCE LENGTH : 34

SEQUENCE TYPE : nucleic acid

STRANDEDNESS : single

TOPOLOGY : linear

MOLECULE TYPE : Other nucleic acid synthetic DNA

SEQUENCE DESCRIPTION:

GGAATTCCAT ATGAGCGCCA AGTCCAGAAC CATA 34

SEQ ID NO : 6

SEQUENCE LENGTH : 36

SEQUENCE TYPE : nucleic acid

STRANDEDNESS : single

TOPOLOGY : linear

MOLECULE TYPE : Other nucleic acid synthetic DNA

SEQUENCE DESCRIPTION:

CCGGGTCGAC TTACTTAGGT GGGTTAAGGT AGTCAA 36

SEQ ID NO : 7

SEQUENCE LENGTH : 9 6 9

SEQUENCE TYPE : nucleic acid

STRANDEDNESS : single

TOPOLOGY : linear

MOLECULE TYPE : D N A

ORIGINAL SOURCE

ORGANISM : Homo sapiens

TISSUE TYPE : liver

SEQUENCE DESCRIPTION:

```
ATG AGC GCC AAG TCC AGA ACC ATA GGG ATT ATT GGA GCT CCT TTC TCA   48
Met Ser Ala Lys Ser Arg Thr Ile Gly Ile Ile Gly Ala Pro Phe Ser
  1               5                  10                  15
AAG GGA CAG CCA CGA GGA GGG GTG GAA GAA GGC CCT ACA GTA TTG AGA   96
Lys Gly Gln Pro Arg Gly Gly Val Glu Glu Gly Pro Thr Val Leu Arg
             20                  25                  30
AAG GCT GGT CTG CTT GAG AAA CTT AAA GAA CAA GAG TGT GAT GTG AAG  144
Lys Ala Gly Leu Leu Glu Lys Leu Lys Glu Gln Glu Cys Asp Val Lys
         35                  40                  45
GAT TAT GGG GAC CTG CCC TTT GCT GAC ATA CCT AAT GAC AGT CCC TTT  192
Asp Tyr Gly Asp Leu Pro Phe Ala Asp Ile Pro Asn Asp Ser Pro Phe
```

```
    50                  55                  60
CAA ATT GTG AAG AAT CCA AGG TCT GTG GGA AAA GCA AGC GAG CAG CTG  240
Gln Ile Val Lys Asn Pro Arg Ser Val Gly Lys Ala Ser Glu Gln Leu
 65                  70                  75                  80
GCT GGC AAG GTG GCA GAA GTC AAG AAG AAT GGA AGA ATC AGC CTG GTG  288
Ala Gly Lys Val Ala Glu Val Lys Lys Asn Gly Arg Ile Ser Leu Val
                 85                  90                  95
CTG GGC GGA GAC CAC AGT TTG GCA ATT GGA AGC ATC TCT GGC CAT GCC  336
Leu Gly Gly Asp His Ser Leu Ala Ile Gly Ser Ile Ser Gly His Ala
            100                 105                 110
AGG GTC CAC CCT GAT CTT GGA GTC ATC TGG GTG GAT GCT CAC ACT GAT  384
Arg Val His Pro Asp Leu Gly Val Ile Trp Val Asp Ala His Thr Asp
        115                 120                 125
ATC AAC ACT CCA CTG ACA ACC ACA AGT GGA AAC TTG CAT GGA CAA CCT  432
Ile Asn Thr Pro Leu Thr Thr Thr Ser Gly Asn Leu His Gly Gln Pro
    130                 135                 140
GTA TCT TTC CTC CTG AAG GAA CTA AAA GGA AAG ATT CCC GAT GTG CCA  480
Val Ser Phe Leu Leu Lys Glu Leu Lys Gly Lys Ile Pro Asp Val Pro
145                 150                 155                 160
GGA TTC TCC TGG GTG ACT CCC TGT ATA TCT GCC AAG GAT ATT GTG TAT  528
Gly Phe Ser Trp Val Thr Pro Cys Ile Ser Ala Lys Asp Ile Val Tyr
                165                 170                 175
ATT GGC TTG AGA GAC GTG GAC CCT GGG GAA CAC TAC ATT TTG AAA ACT  576
Ile Gly Leu Arg Asp Val Asp Pro Gly Glu His Tyr Ile Leu Lys Thr
            180                 185                 190
CTA GGC ATT AAA TAC TTT TCA ATG ACT GAA GTG GAC AGA CTA GGA ATT  624
Leu Gly Ile Lys Tyr Phe Ser Met Thr Glu Val Asp Arg Leu Gly Ile
        195                 200                 205
GGC AAG GTG ATG GAA GAA ACA CTC AGC TAT CTA CTA GGA AGA AAG AAA  672
```

Gly Lys Val Met Glu Glu Thr Leu Ser Tyr Leu Leu Gly Arg Lys Lys
210 215 220

AGG CCA ATT CAT CTA AGT TTT GAT GTT GAC GGA CTG GAC CCA TCT TTC 720
Arg Pro Ile His Leu Ser Phe Asp Val Asp Gly Leu Asp Pro Ser Phe
225 230 235 240

ACA CCA GCT ACT GGC ACA CCA GTC GTG GGA GGT CTG ACA TAC AGA GAA 768
Thr Pro Ala Thr Gly Thr Pro Val Val Gly Gly Leu Thr Tyr Arg Glu
245 250 255

GGT CTC TAC ATC ACA GAA GAA ATC TAC AAA ACA GGG CTA CTC TCA GGA 816
Gly Leu Tyr Ile Thr Glu Glu Ile Tyr Lys Thr Gly Leu Leu Ser Gly
260 265 270

TTA GAT ATA ATG GAA GTG AAC CCA TCC CTG GGG AAG ACA CCA GAA GAA 864
Leu Asp Ile Met Glu Val Asn Pro Ser Leu Gly Lys Thr Pro Glu Glu
275 280 285

GTA ACT CGA ACA GTG AAC ACA GCA GTT GCA ATA ACC TTG GCT TGT TTC 912
Val Thr Arg Thr Val Asn Thr Ala Val Ala Ile Thr Leu Ala Cys Phe
290 295 300

GGA CTT GCT CGG GAG GGT AAT CAC AAG CCT ATT GAC TAC CTT AAC CCA 960
Gly Leu Ala Arg Glu Gly Asn His Lys Pro Ile Asp Tyr Leu Asn Pro
305 310 315 320

CCT AAG TAA 969
Pro Lys

SEQ ID NO: 8
SEQUENCE LENGTH: 322
SEQUENCE TYPE: amino acid
STRANDEDNESS: single
TOPOLOGY: linear
MOLECULE TYPE: peptide

ORIGINAL SOURCE

ORGANISM: Homo sapiens

TISSUE TYPE: liver

SEQUENCE DESCRIPTION:

Met Ser Ala Lys Ser Arg Thr Ile Gly Ile Ile Gly Ala Pro Phe Ser
1 5 10 15

Lys Gly Gln Pro Arg Gly Gly Val Glu Glu Gly Pro Thr Val Leu Arg
20 25 30

Lys Ala Gly Leu Leu Glu Lys Leu Lys Glu Gln Glu Cys Asp Val Lys
35 40 45

Asp Tyr Gly Asp Leu Pro Phe Ala Asp Ile Pro Asn Asp Ser Pro Phe
50 55 60

Gln Ile Val Lys Asn Pro Arg Ser Val Gly Lys Ala Ser Glu Gln Leu
65 70 75 80

Ala Gly Lys Val Ala Glu Val Lys Lys Asn Gly Arg Ile Ser Leu Val
85 90 95

Leu Gly Gly Asp His Ser Leu Ala Ile Gly Ser Ile Ser Gly His Ala
100 105 110

Arg Val His Pro Asp Leu Gly Val Ile Trp Val Asp Ala His Thr Asp
115 120 125

Ile Asn Thr Pro Leu Thr Thr Thr Ser Gly Asn Leu His Gly Gln Pro
130 135 140

Val Ser Phe Leu Leu Lys Glu Leu Lys Gly Lys Ile Pro Asp Val Pro
145 150 155 160

Gly Phe Ser Trp Val Thr Pro Cys Ile Ser Ala Lys Asp Ile Val Tyr
165 170 175

Ile Gly Leu Arg Asp Val Asp Pro Gly Glu His Tyr Ile Leu Lys Thr
180 185 190

Leu Gly Ile Lys Tyr Phe Ser Met Thr Glu Val Asp Arg Leu Gly Ile

195 200 205

Gly Lys Val Met Glu Glu Thr Leu Ser Tyr Leu Leu Gly Arg Lys Lys

210 215 220

Arg Pro Ile His Leu Ser Phe Asp Val Asp Gly Leu Asp Pro Ser Phe

225 230 235 240

Thr Pro Ala Thr Gly Thr Pro Val Val Gly Gly Leu Thr Tyr Arg Glu

245 250 255

Gly Leu Tyr Ile Thr Glu Glu Ile Tyr Lys Thr Gly Leu Leu Ser Gly

260 265 270

Leu Asp Ile Met Glu Val Asn Pro Ser Leu Gly Lys Thr Pro Glu Glu

275 280 285

Val Thr Arg Thr Val Asn Thr Ala Val Ala Ile Thr Leu Ala Cys Phe

290 295 300

Gly Leu Ala Arg Glu Gly Asn His Lys Pro Ile Asp Tyr Leu Asn Pro

305 310 315 320

ProLys

SEQ ID NO: 9

SEQUENCE LENGTH : 972

SEQUENCE TYPE: nucleic acid

STRANDEDNESS: single

TOPOLOGY: linear

MOLECULE TYPE : DNA

ORIGINAL SOURCE

ORGANISM: Rattus norvegicus

TISSUE TYPE: liver

SEQUENCE DESCRIPTION:

ATG AGC TCC AAG CCA AAG CCC ATA GAG ATT ATC GGA GCG CCT TTC TCT 48

Met Ser Ser Lys Pro Lys Pro Ile Glu Ile Ile Gly Ala Pro Phe Ser

1 5 10 15

AAG GGA CAG CCT CGA GGA GGG GTA GAG AAA GGT CCC GCA GCA TTA AGG 96

Lys Gly Gln Pro Arg Gly Gly Val Glu Lys Gly Pro Ala Ala Leu Arg

20 25 30

AAA GCT GGC CTG GTG GAG AAG CTT AAA GAA ACA GAG TAC AAT GTG AGA 144

Lys Ala Gly Leu Val Glu Lys Leu Lys Glu Thr Glu Tyr Asn Val Arg

35 40 45

GAC CAC GGG GAT CTG GCC TTT GTG GAT GTC CCC AAT GAC AGC CCC TTT 192

Asp His Gly Asp Leu Ala Phe Val Asp Val Pro Asn Asp Ser Pro Phe

50 55 60

CAA ATT GTG AAG AAC CCA CGG TCT GTG GGA AAA GCC AAT GAA CAG CTG 240

Gln Ile Val Lys Asn Pro Arg Ser Val Gly Lys Ala Asn Glu Gln Leu

65 70 75 80

GCT GCT GTG GTA GCG GAG ACC CAG AAG AAT GGA ACA ATC AGT GTG GTG 288

Ala Ala Val Val Ala Glu Thr Gln Lys Asn Gly Thr Ile Ser Val Val

85 90 95

CTG GGT GGA GAC CAC AGT ATG GCA ATT GGA AGC ATC TCT GGC CAC GCC 336

Leu Gly Gly Asp His Ser Met Ala Ile Gly Ser Ile Ser Gly His Ala

100 105 110

AGG GTC CAC CCT GAC CTA TGC GTC ATT TGG GTG GAT GCT CAC ACT GAC 384

Arg Val His Pro Asp Leu Cys Val Ile Trp Val Asp Ala His Thr Asp

115 120 125

ATC AAC ACT CCG CTG ACA ACC AGC TCT GGG AAT CTG CAC GGG CAA CCG 432

Ile Asn Thr Pro Leu Thr Thr Ser Ser Gly Asn Leu His Gly Gln Pro

130 135 140

GTG GCC TTT CTC CTG AAG GAA CTG AAA GGA AAG TTC CCA GAT GTA CCA 480

Val Ala Phe Leu Leu Lys Glu Leu Lys Gly Lys Phe Pro Asp Val Pro

145 150 155 160

```
GGA TTC TCC TGG GTG ACC CCC TGC ATA TCT GCC AAG GAC ATC GTG TAC  528
Gly Phe Ser Trp Val Thr Pro Cys Ile Ser Ala Lys Asp Ile Val Tyr
                165                 170                 175
ATC GGC TTG CGA GAT GTG GAC CCT GGG GAA CAC TAT ATA ATA AAA ACT  576
Ile Gly Leu Arg Asp Val Asp Pro Gly Glu His Tyr Ile Ile Lys Thr
            180                 185                 190
CTG GGC ATT AAG TAT TTC TCA ATG ACT GAA GTG GAC AAG CTG GGA ATT  624
Leu Gly Ile Lys Tyr Phe Ser Met Thr Glu Val Asp Lys Leu Gly Ile
        195                 200                 205
GGC AAA GTG ATG GAA GAG ACC TTC AGC TAC CTG CTG GGA AGG AAG AAA  672
Gly Lys Val Met Glu Glu Thr Phe Ser Tyr Leu Leu Gly Arg Lys Lys
    210                 215                 220
AGG CCC ATT CAC CTG AGT TTT GAT GTT GAT GGA CTG GAC CCA GTA TTC  720
Arg Pro Ile His Leu Ser Phe Asp Val Asp Gly Leu Asp Pro Val Phe
225                 230                 235                 240
ACC CCG GCT ACG GGC ACA CCC GTT GTG GGA GGC CTA TCT TAC AGA GAA  768
Thr Pro Ala Thr Gly Thr Pro Val Val Gly Gly Leu Ser Tyr Arg Glu
                245                 250                 255
GGT CTC TAC ATC ACA GAA GAA ATT TAC AAG ACA GGG CTA CTT TCA GGA  816
Gly Leu Tyr Ile Thr Glu Glu Ile Tyr Lys Thr Gly Leu Leu Ser Gly
            260                 265                 270
CTA GAT ATC ATG GAA GTG AAC CCA ACT CTT GGG AAG ACA CCA GAG GAG  864
Leu Asp Ile Met Glu Val Asn Pro Thr Leu Gly Lys Thr Pro Glu Glu
        275                 280                 285
GTG ACT CGT ACT GTG AAC ACG GCA GTG GCG TTG ACC TTG TCT TGT TTT  912
Val Thr Arg Thr Val Asn Thr Ala Val Ala Leu Thr Leu Ser Cys Phe
    290                 295                 300
GGA ACG AAA CGG GAA GGT AAT CAT AAG CCA GAG ACT GAC TAC CTT AAA  960
Gly Thr Lys Arg Glu Gly Asn His Lys Pro Glu Thr Asp Tyr Leu Lys
```

305 310 315 320

CCA CCG AAA TAA 972

Pro Pro Lys

SEQ ID NO: 10

SEQUENCE LENGTH : 323

SEQUENCE TYPE: amino acid

STRANDEDNESS: single

TOPOLOGY: linear

MOLECULE TYPE: peptide

ORIGINAL SOURCE

ORGANISM: Rattus norvegicus

TISSUE TYPE: liver

SEQUENCE DESCRIPTION:

Met Ser Ser Lys Pro Lys Pro Ile Glu Ile Ile Gly Ala Pro Phe Ser

1 5 10 15

Lys Gly Gln Pro Arg Gly Gly Val Glu Lys Gly Pro Ala Ala Leu Arg

20 25 30

Lys Ala Gly Leu Val Glu Lys Leu Lys Glu Thr Glu Tyr Asn Val Arg

35 40 45

Asp His Gly Asp Leu Ala Phe Val Asp Val Pro Asn Asp Ser Pro Phe

50 55 60

Gln Ile Val Lys Asn Pro Arg Ser Val Gly Lys Ala Asn Glu Gln Leu

65 70 75 80

Ala Ala Val Val Ala Glu Thr Gln Lys Asn Gly Thr Ile Ser Val Val

85 90 95

Leu Gly Gly Asp His Ser Met Ala Ile Gly Ser Ile Ser Gly His Ala

100 105 110

Arg Val His Pro Asp Leu Cys Val Ile Trp Val Asp Ala His Thr Asp

115 120 125

Ile Asn Thr Pro Leu Thr Thr Ser Ser Gly Asn Leu His Gly Gln Pro

130 135 140

Val Ala Phe Leu Leu Lys Glu Leu Lys Gly Lys Phe Pro Asp Val Pro

145 150 155 160

Gly Phe Ser Trp Val Thr Pro Cys Ile Ser Ala Lys Asp Ile Val Tyr

165 170 175

Ile Gly Leu Arg Asp Val Asp Pro Gly Glu His Tyr Ile Ile Lys Thr

180 185 190

Leu Gly Ile Lys Tyr Phe Ser Met Thr Glu Val Asp Lys Leu Gly Ile

195 200 205

Gly Lys Val Met Glu Glu Thr Phe Ser Tyr Leu Leu Gly Arg Lys Lys

210 215 220

Arg Pro Ile His Leu Ser Phe Asp Val Asp Gly Leu Asp Pro Val Phe

225 230 235 240

Thr Pro Ala Thr Gly Thr Pro Val Val Gly Gly Leu Ser Tyr Arg Glu

245 250 255

Gly Leu Tyr Ile Thr Glu Glu Ile Tyr Lys Thr Gly Leu Leu Ser Gly

260 265 270

Leu Asp Ile Met Glu Val Asn Pro Thr Leu Gly Lys Thr Pro Glu Glu

275 280 285

Val Thr Arg Thr Val Asn Thr Ala Val Ala Leu Thr Leu Ser Cys Phe

290 295 300

Gly Thr Lys Arg Glu Gly Asn His Lys Pro Glu Thr Asp Tyr Leu Lys

305 310 315 320

Pro Pro Lys

SEQ ID NO : 11

SEQUENCE LENGTH : 34

SEQUENCE TYPE : nucleic acid

STRANDEDNESS : single

TOPOLOGY : linear

MOLECULE TYPE : Other nucleic acid synthetic DNA

SEQUENCE DESCRIPTION:

GGAATTCCAT ATGAGCTCCA AGCCAAAGCC CATA 34

SEQ ID NO : 1 2

SEQUENCE LENGTH : 3 6

SEQUENCE TYPE : nucleic acid

STRANDEDNESS : single

TOPOLOGY : linear

MOLECULE TYPE : Other nucleic acid synthetic DNA

SEQUENCE DESCRIPTION:

CCGGGTCGAC TTATTTCGGT GGTTTAAGGT AGTCAG 36

## Claims

1. A tissue fibrosis inhibiting agent comprising an arginine-degrading enzyme, a variant or a modified derivative thereof as an active ingredient.

2. The tissue fibrosis inhibiting agent of claim 1, wherein the arginine-degrading enzyme is arginase.

3. The tissue fibrosis inhibiting agent of claim 2, wherein the arginase is a human or rat arginase.

4. The tissue fibrosis inhibiting agent of claim 3, wherein the rat arginase is extracted from a rat liver and subsequently purified.

5. The tissue fibrosis inhibiting agent of claim 1, wherein the arginine-degrading enzyme is arginine deiminase.

6. The tissue fibrosis inhibiting agent of claim 1, wherein the modified derivative is arginase modified with polyethylene glycol.

7. The tissue fibrosis inhibiting agent of claim 1, wherein the tissue is the liver, kidney, lung, pancreas or skin.

8. A DNA molecule comprising the nucleotide sequence of SEQ ID NO:7.

9. A DNA molecule which hybridizes with the nucleotide sequence of SEQ ID NO:7 under stringent conditions and which encodes a human arginase having a tissue fibrosis inhibiting activity.

10. A recombinant vector containing the DNA molecule of claim 8 or 9.

11. A transformant obtained by introducing the recombinant vector of claim 10 into a host.

12. A method of producing a human arginase, which comprises culturing the transformant of claim 11 in a medium to produce and accumulate a human arginase in the culture solution and recovering the human arginase from the culture solution.

13. A DNA molecule comprising nucleotide sequence of SEQ ID NO:9.

14. A DNA molecule which hybridizes with the nucleotide sequence of SEQ ID NO:9 under stringent conditions and which encodes a rat arginase having a tissue fibrosis inhibiting activity.

15. A recombinant vector containing the DNA molecule of claim 13 or 14.

16. A transformant obtained by introducing the recombinant vector of claim 15 into a host.

17. A method of producing a rat arginase, which comprises culturing the transformant of claim 16 in a medium to produce and accumulate a rat arginase in the culture and recovering the rat arginase from the culture.

FIG.1

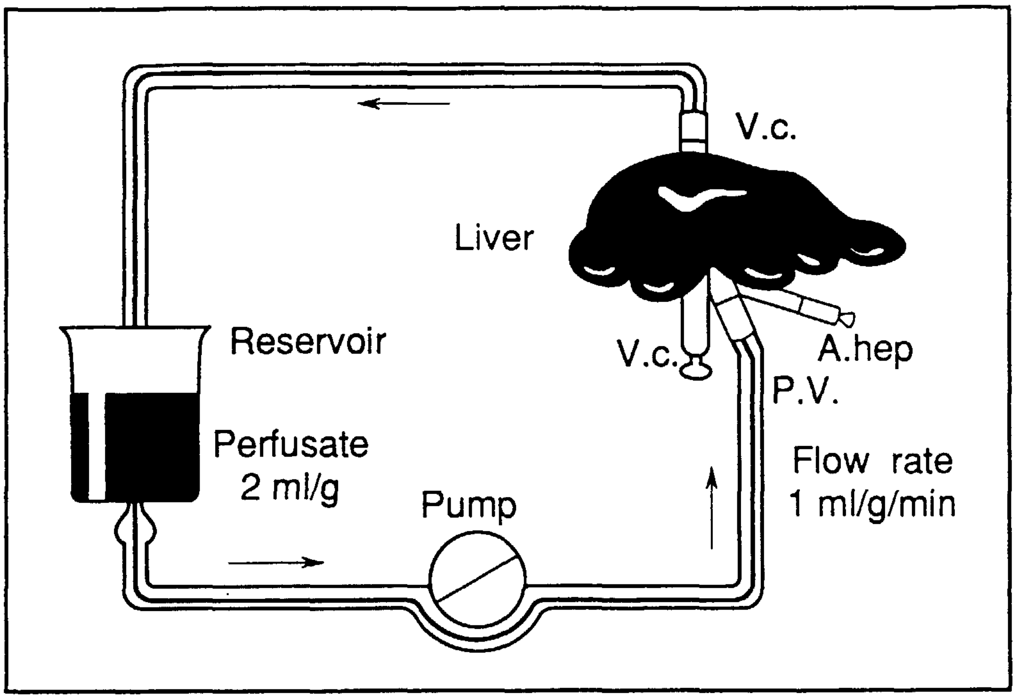
V.c.
Liver
Reservoir
V.c.
A.hep
P.V.
Perfusate
2 ml/g
Pump
Flow rate
1 ml/g/min

FIG.2

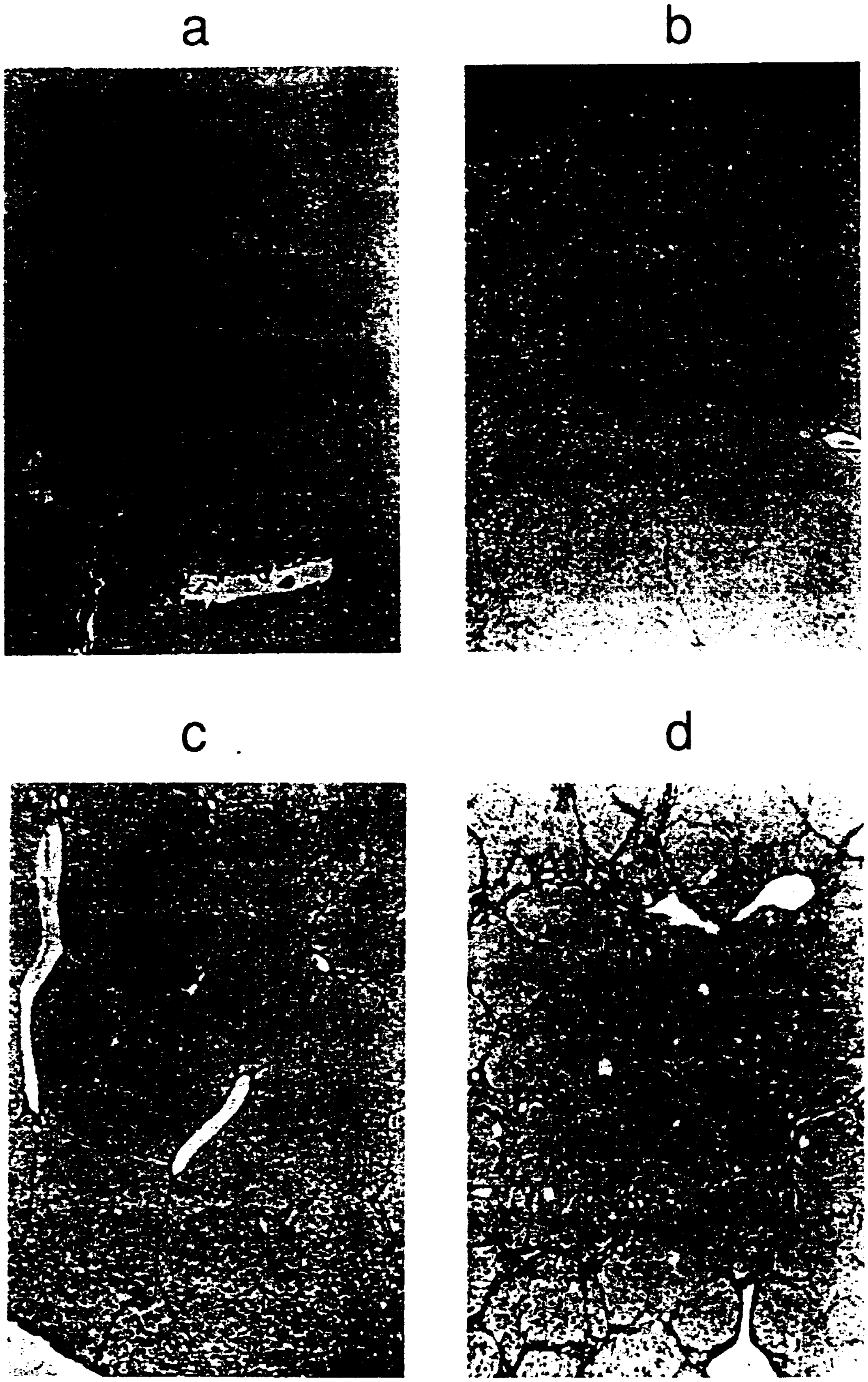

FIG.3

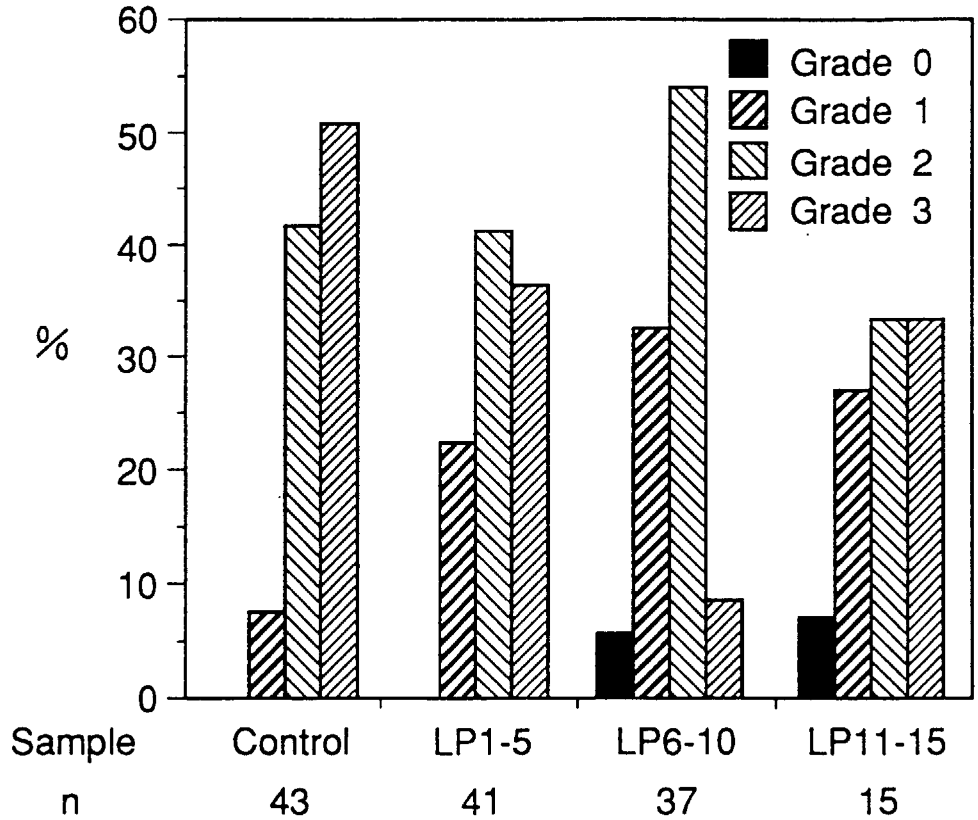
60
50
40
%
30
20
10
0
Grade 0
Grade 1
Grade 2
Grade 3
Sample
Control
LP1-5
LP6-10
LP11-15
n
43
41
37
15

FIG.4

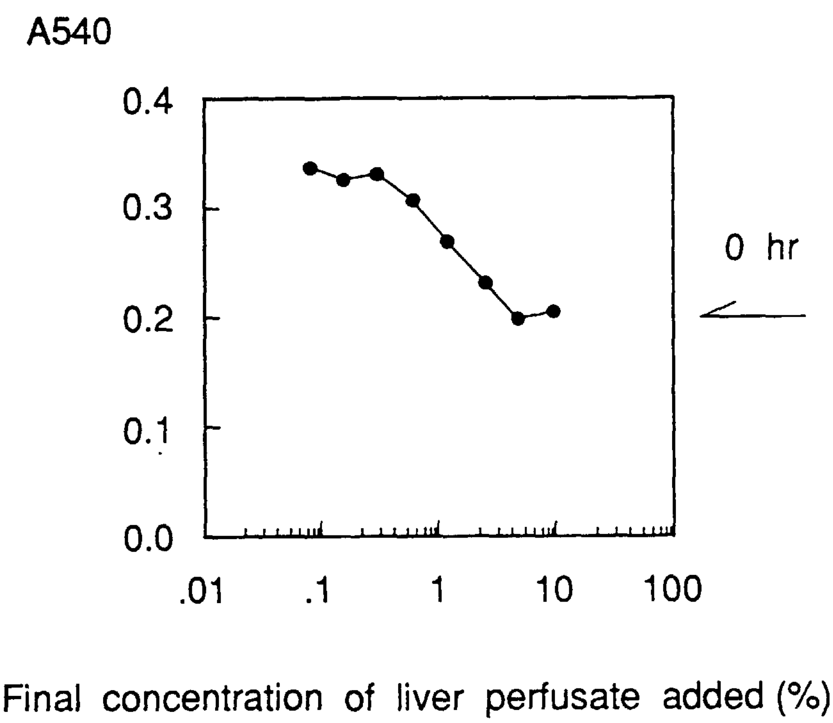
A540
0.4
0.3
0.2
0.1
0.0
.01
.1
1
10
100
0 hr
Final concentration of liver perfusate added (%)

FIG.5

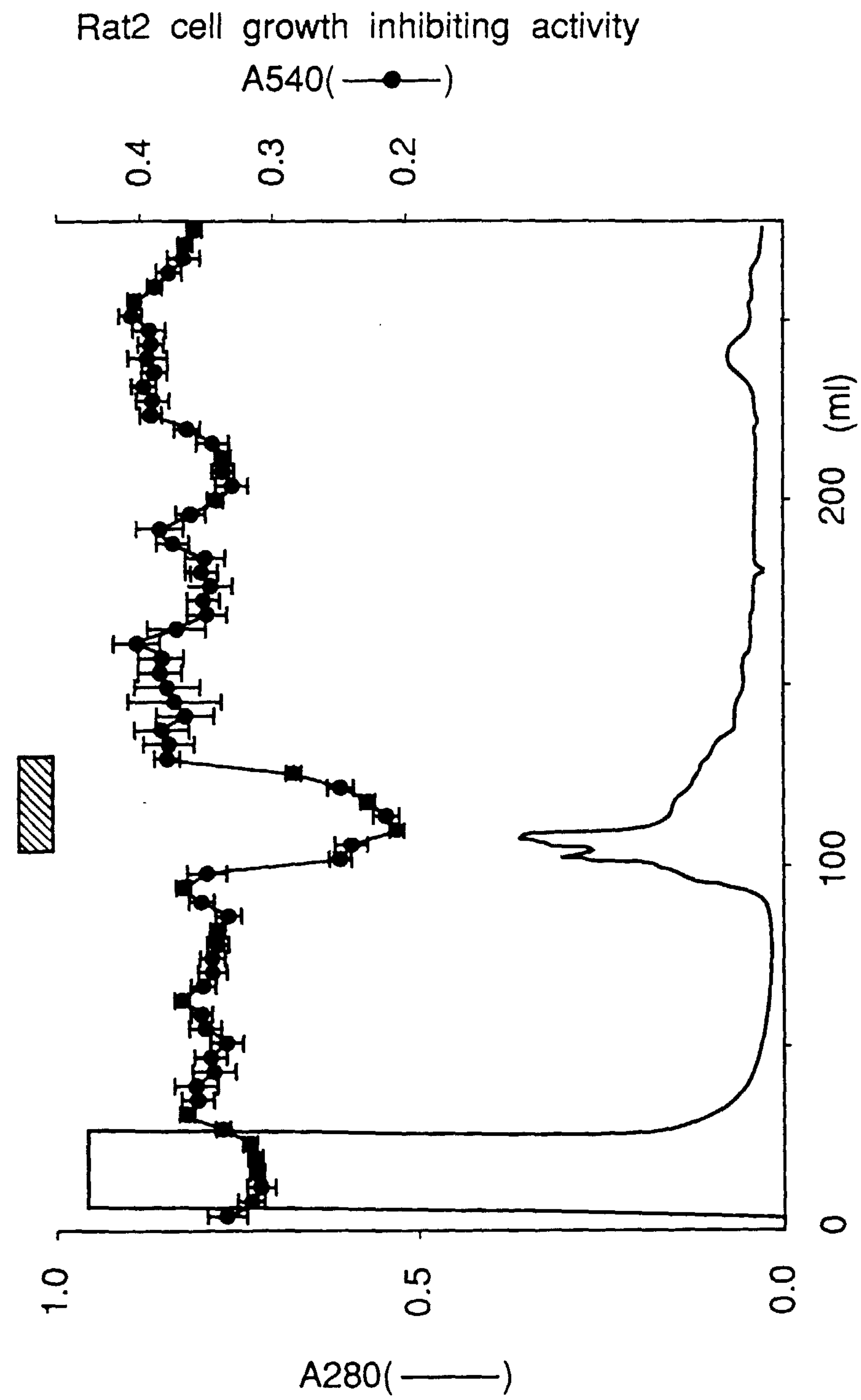
Rat2 cell growth inhibiting activity
A540(—●—)
0.4
0.3
0.2
1.0
0.5
0.0
A280(——)
0
100
200 (ml)

FIG.6

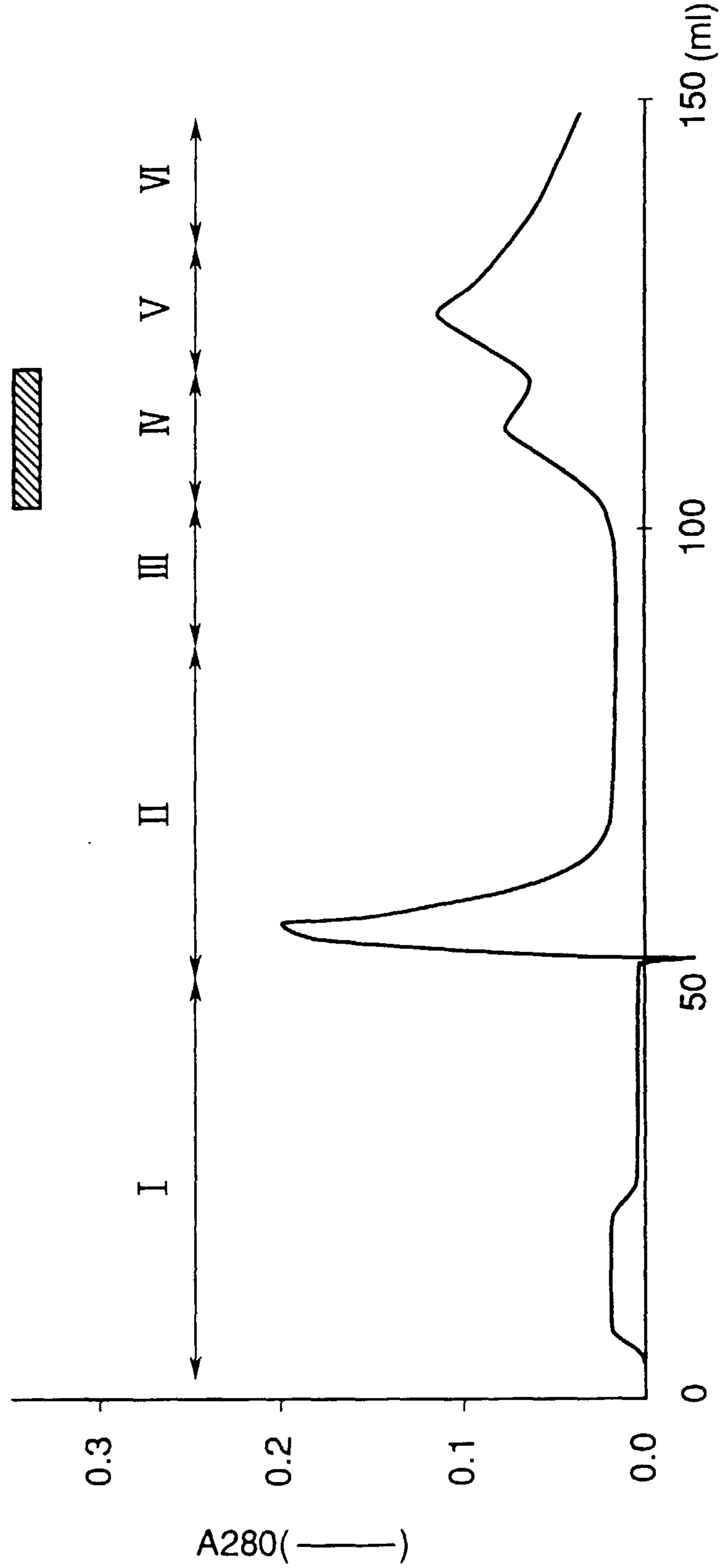

FIG.7

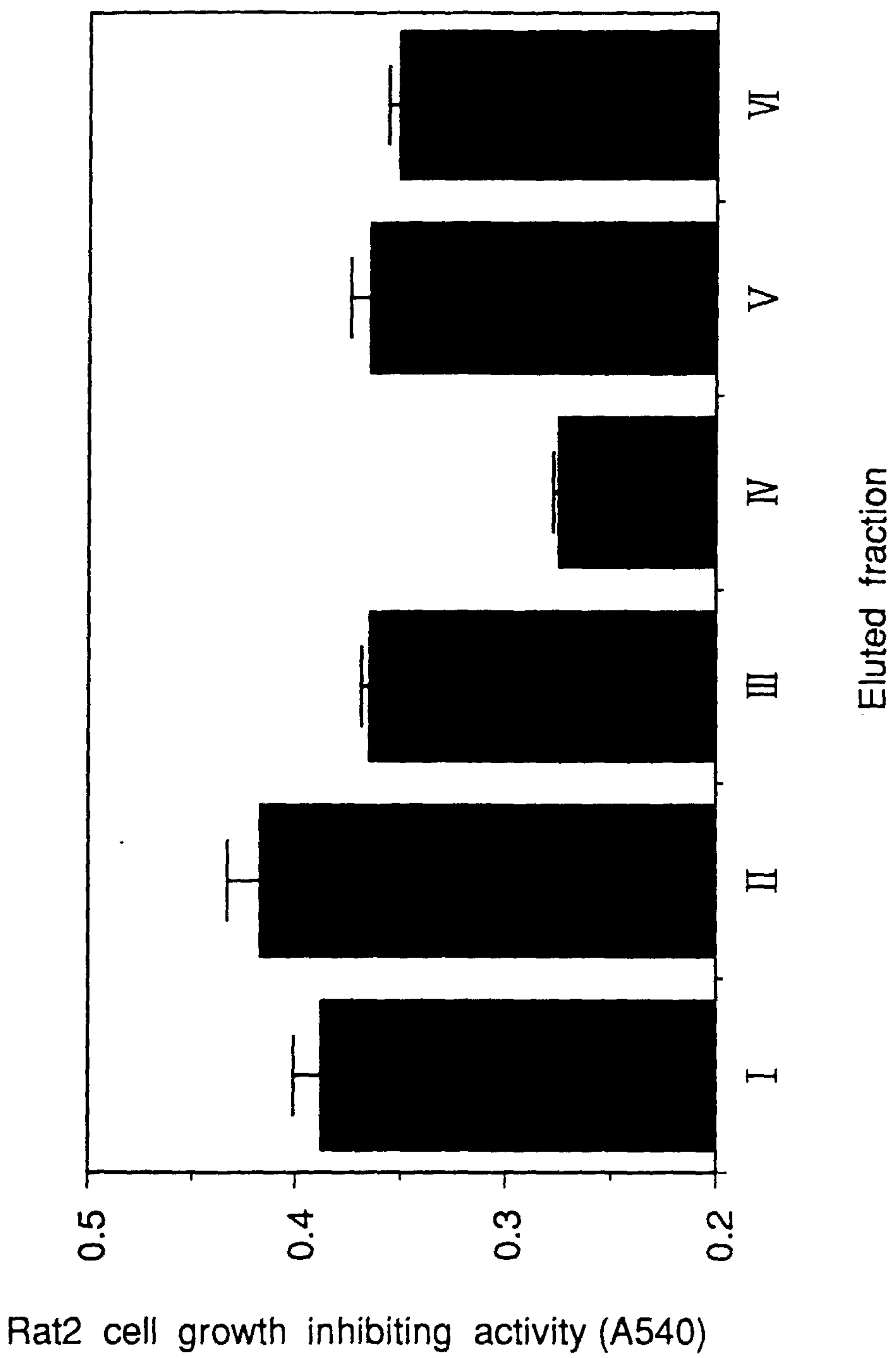
0.5
0.4
0.3
0.2
Rat2 cell growth inhibiting activity (A540)
I
II
III
IV
V
VI
Eluted fraction

FIG.8

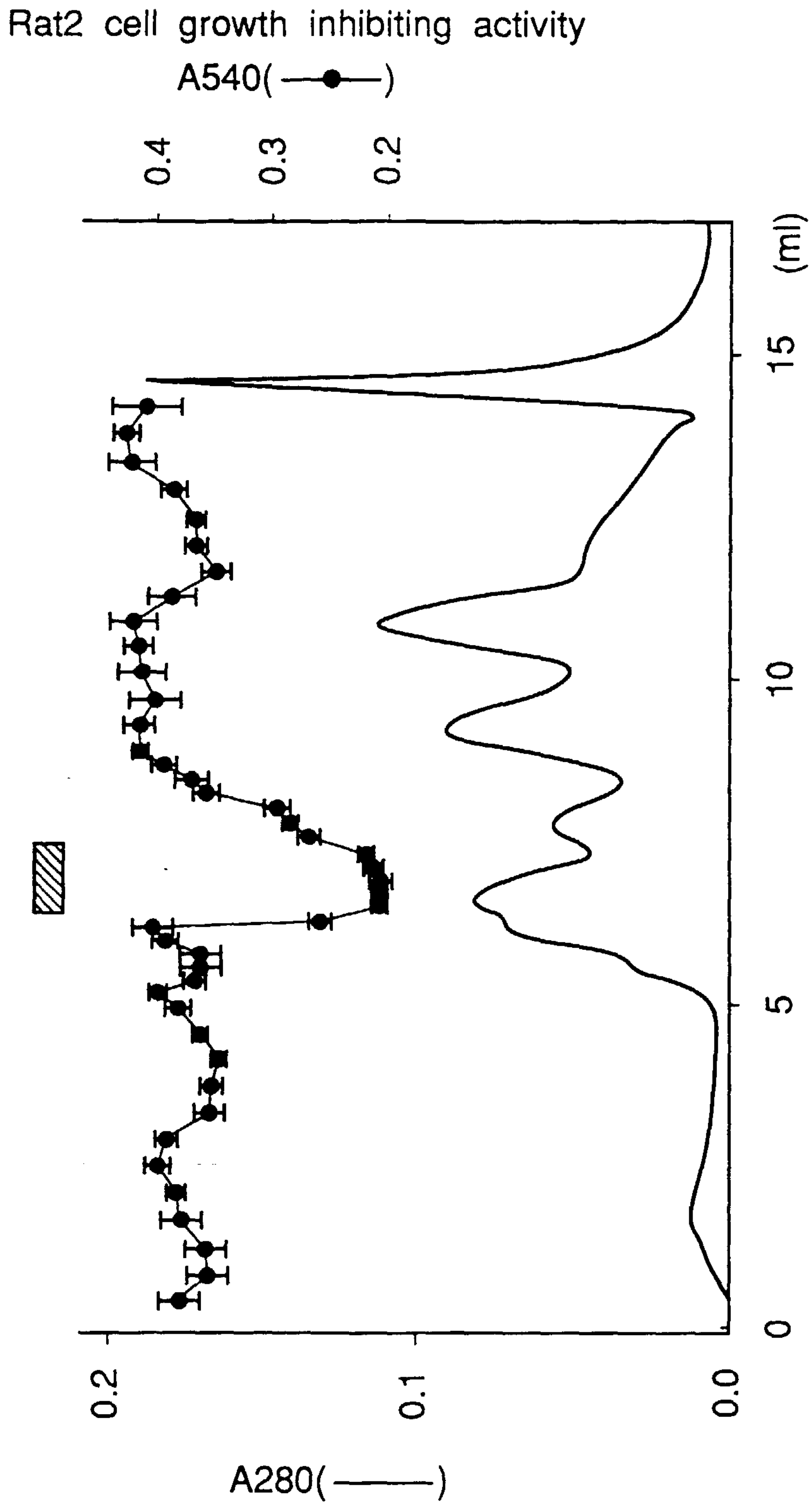
Rat2 cell growth inhibiting activity
A540(—●—)
0.4
0.3
0.2
A280(——)
0.2
0.1
0.0
0
5
10
15
(ml)

FIG.9

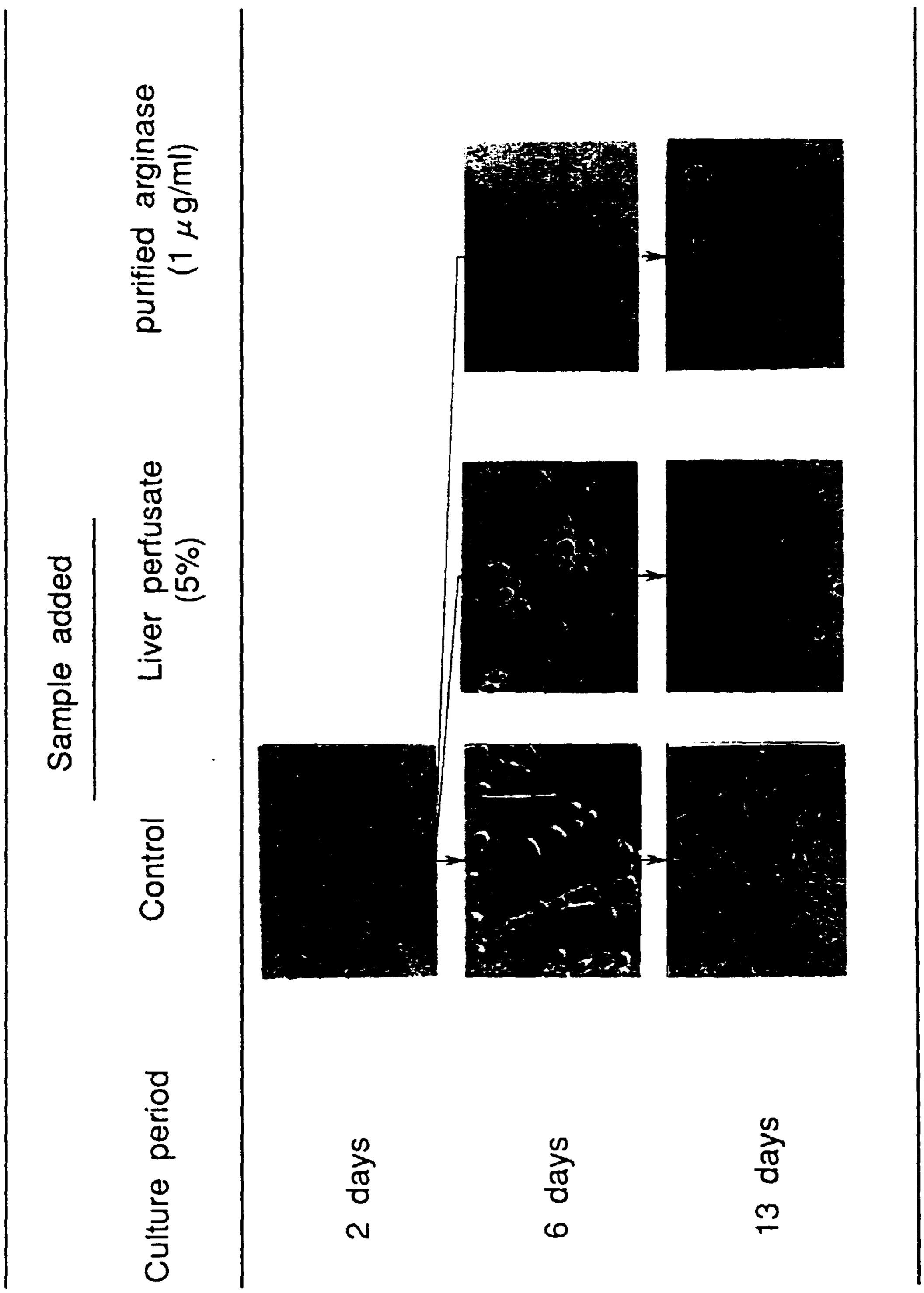
Culture period
Sample added
Control
Liver perfusate
(5%)
purified arginase
(1 μg/ml)
2 days
6 days
13 days

FIG.10

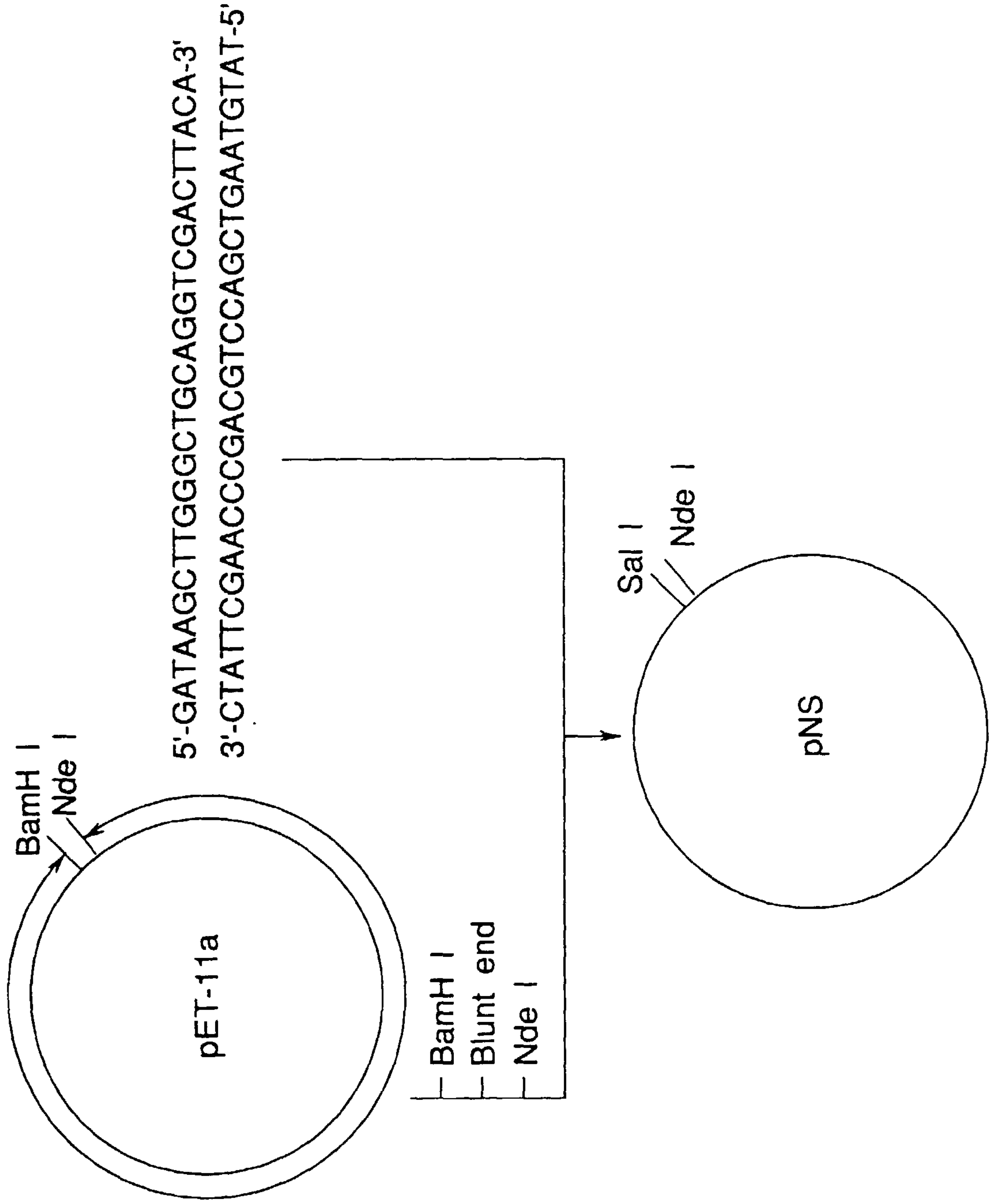
5'-GATAAGCTTGGGCTGCAGGTCGACTTACA-3'
3'-CTATTCGAACCCGACGTCCAGCTGAATGTAT-5'
pET-11a
BamH I
Nde I
BamH I
Blunt end
Nde I
pNS
Sal I
Nde I

FIG.11

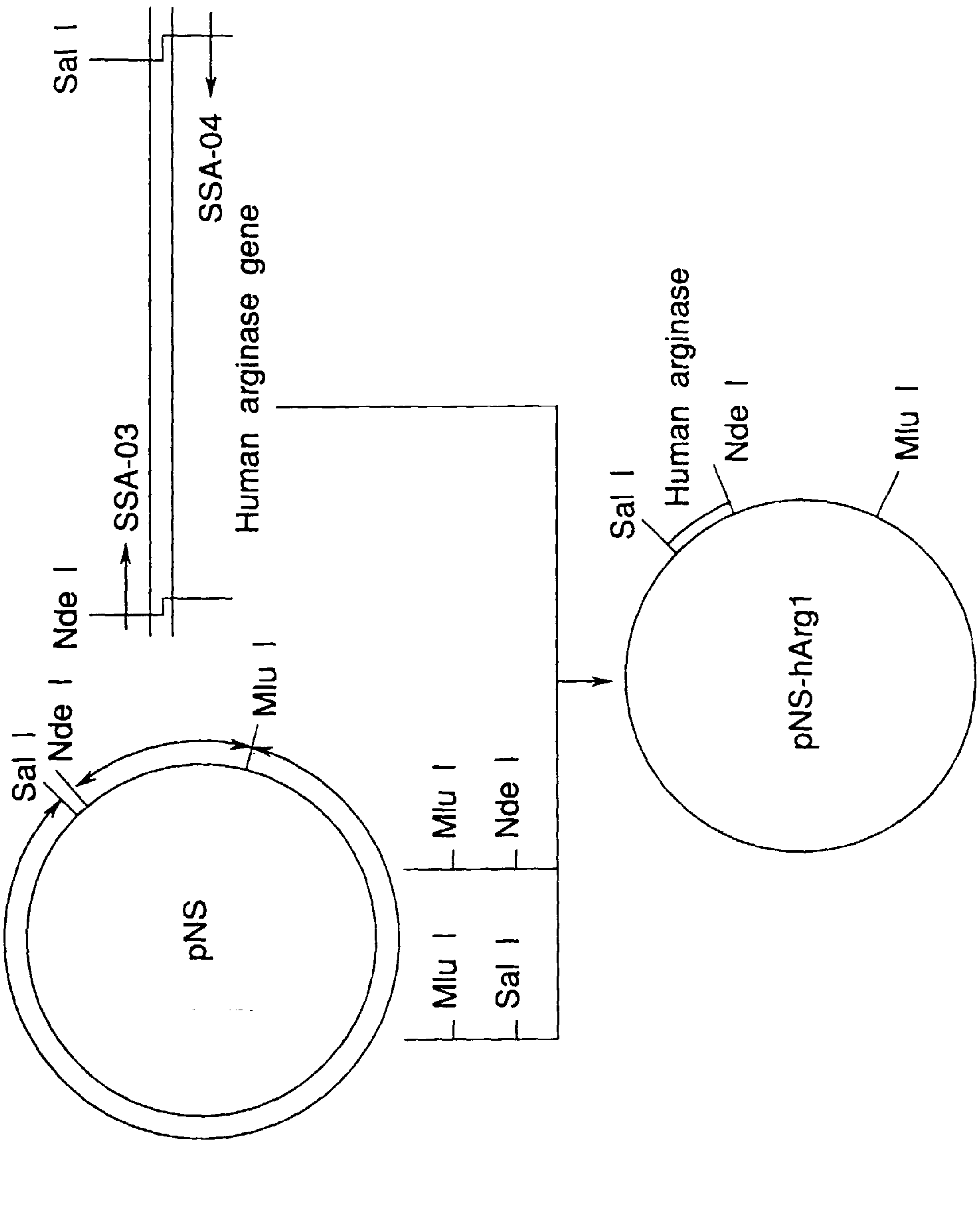
Sal I
Nde I
Nde I
SSA-03
SSA-04
Sal I
Human arginase gene
Mlu I
pNS
Mlu I
Sal I
Mlu I
Nde I
Sal I
Human arginase
Nde I
Mlu I
pNS-hArg1

FIG.12

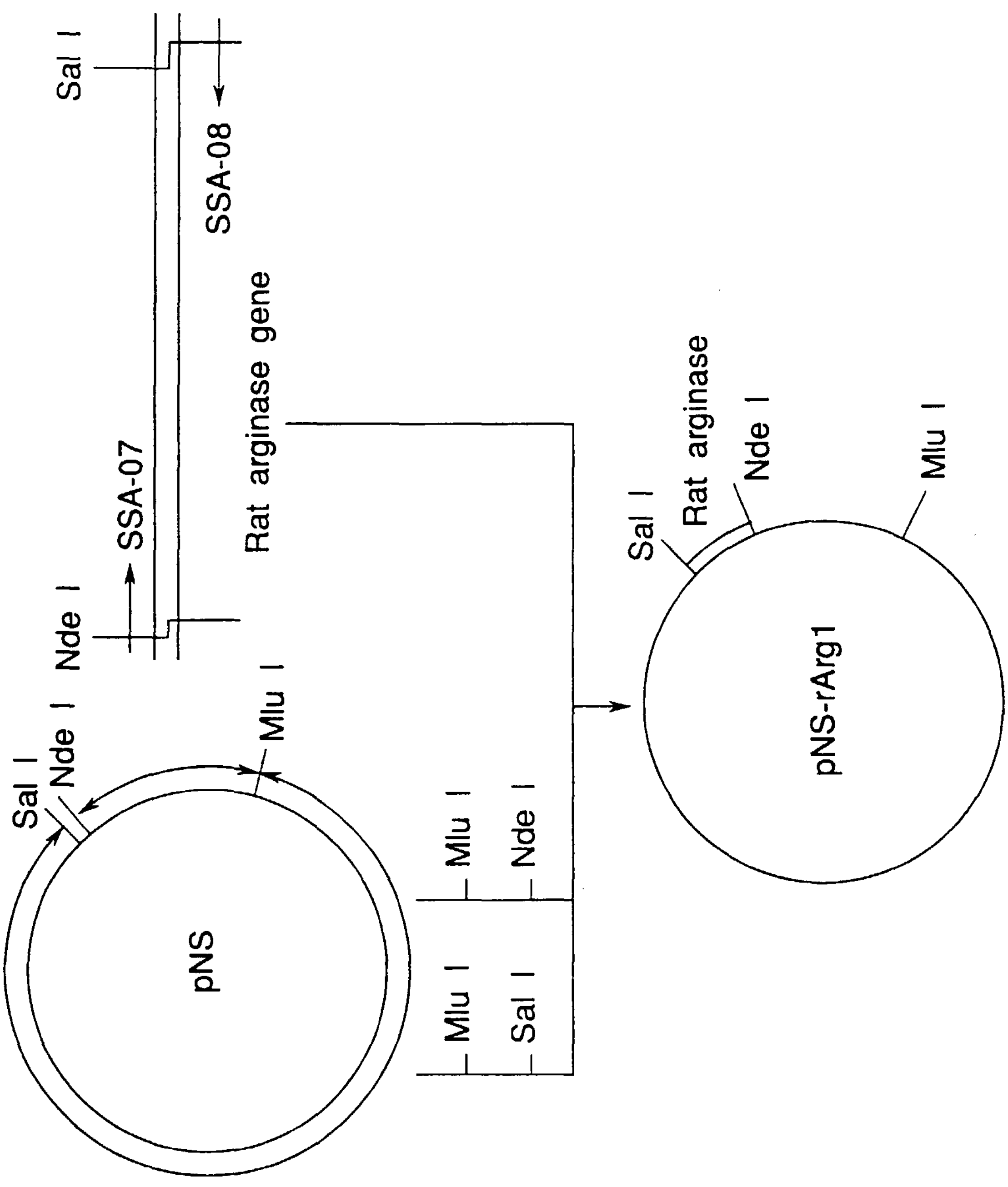

Nde I
SSA-07
Sal I
SSA-08
Rat arginase gene
Sal I
Nde I
Nde I
Mlu I
pNS
Mlu I
Sal I
Mlu I
Nde I
Sal I
Rat arginase
Nde I
Mlu I
pNS-rArg1

FIG.13

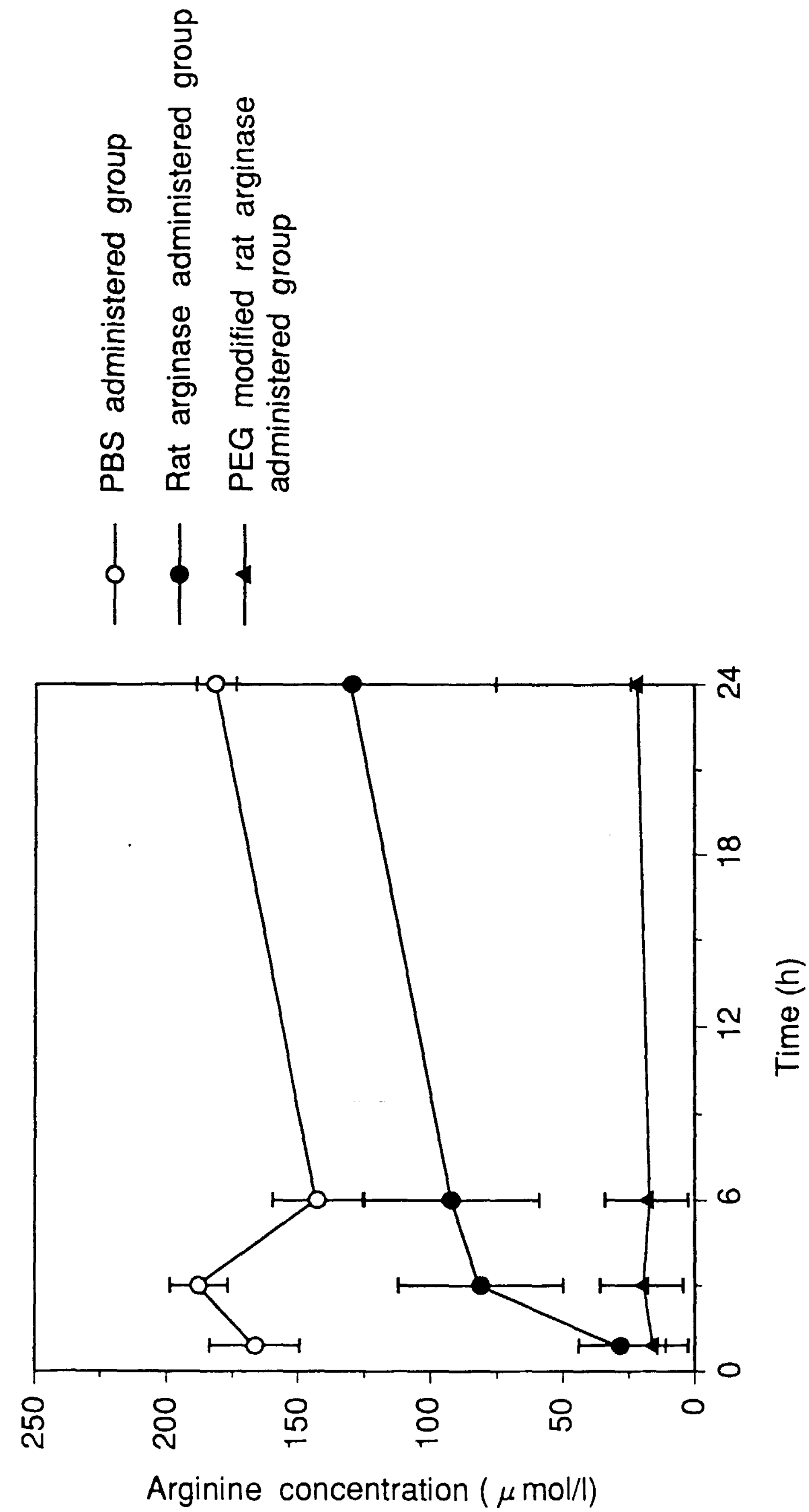
PBS administered group
Rat arginase administered group
PEG modified rat arginase administered group
Arginine concentration ( μ mol/l)
250
200
150
100
50
0
Time (h)
0
6
12
18
24

FIG.14

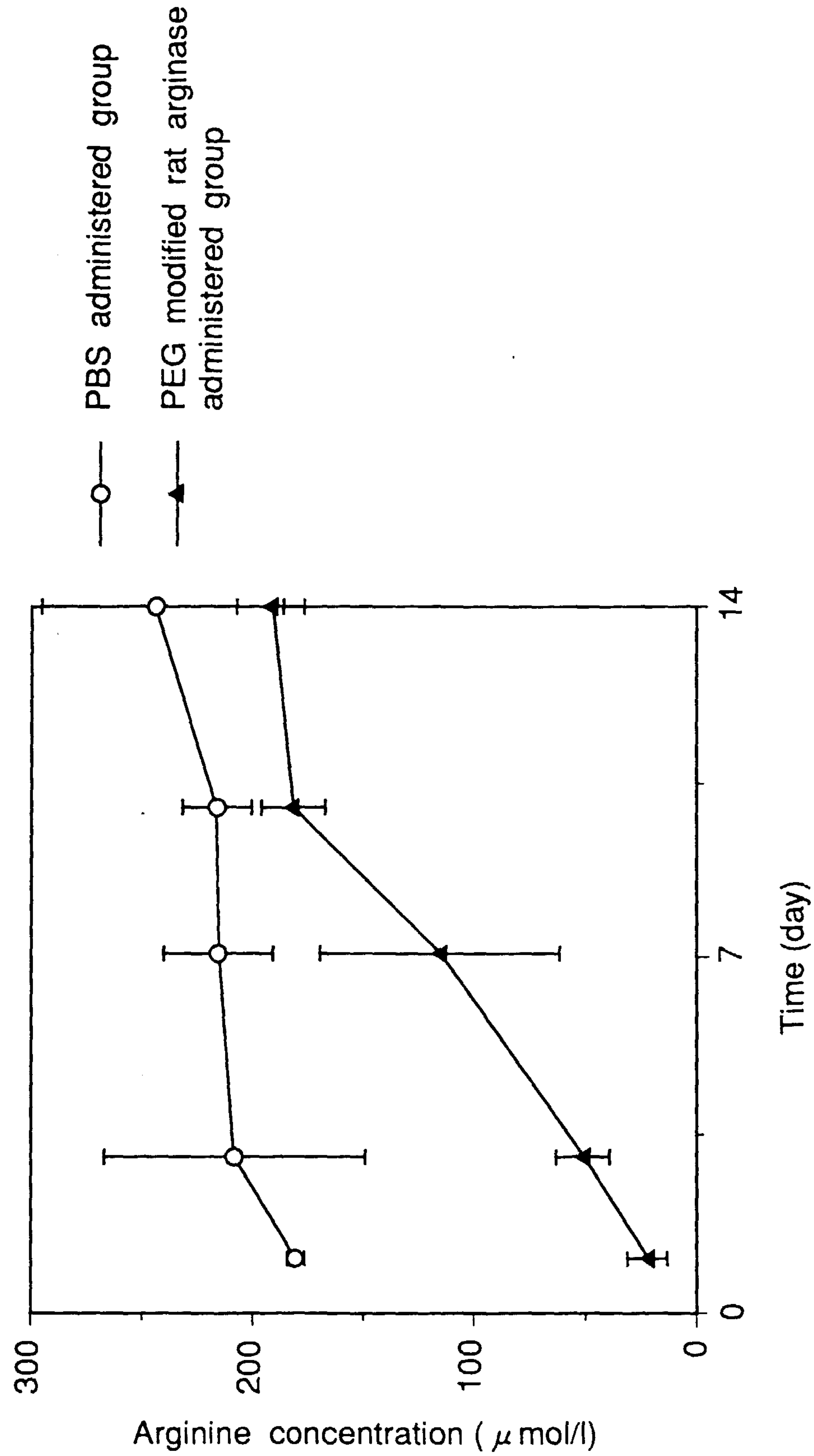
PBS administered group
PEG modified rat arginase administered group
Arginine concentration ( μ mol/l)
300
200
100
0
0
7
14
Time (day)

FIG.15

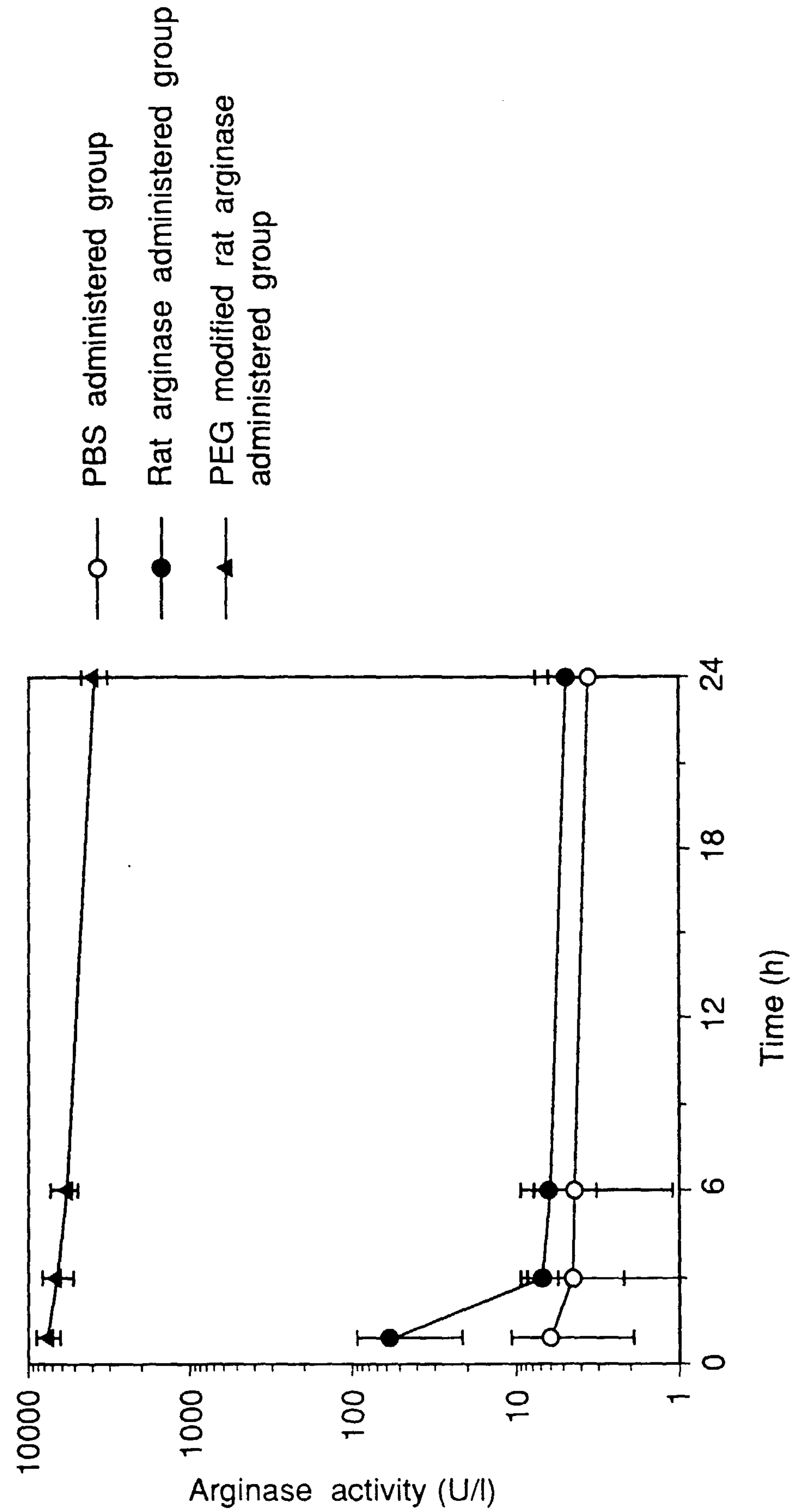
PBS administered group
Rat arginase administered group
PEG modified rat arginase administered group
Arginase activity (U/l)
10000
1000
100
10
1
Time (h)
0
6
12
18
24

FIG.16

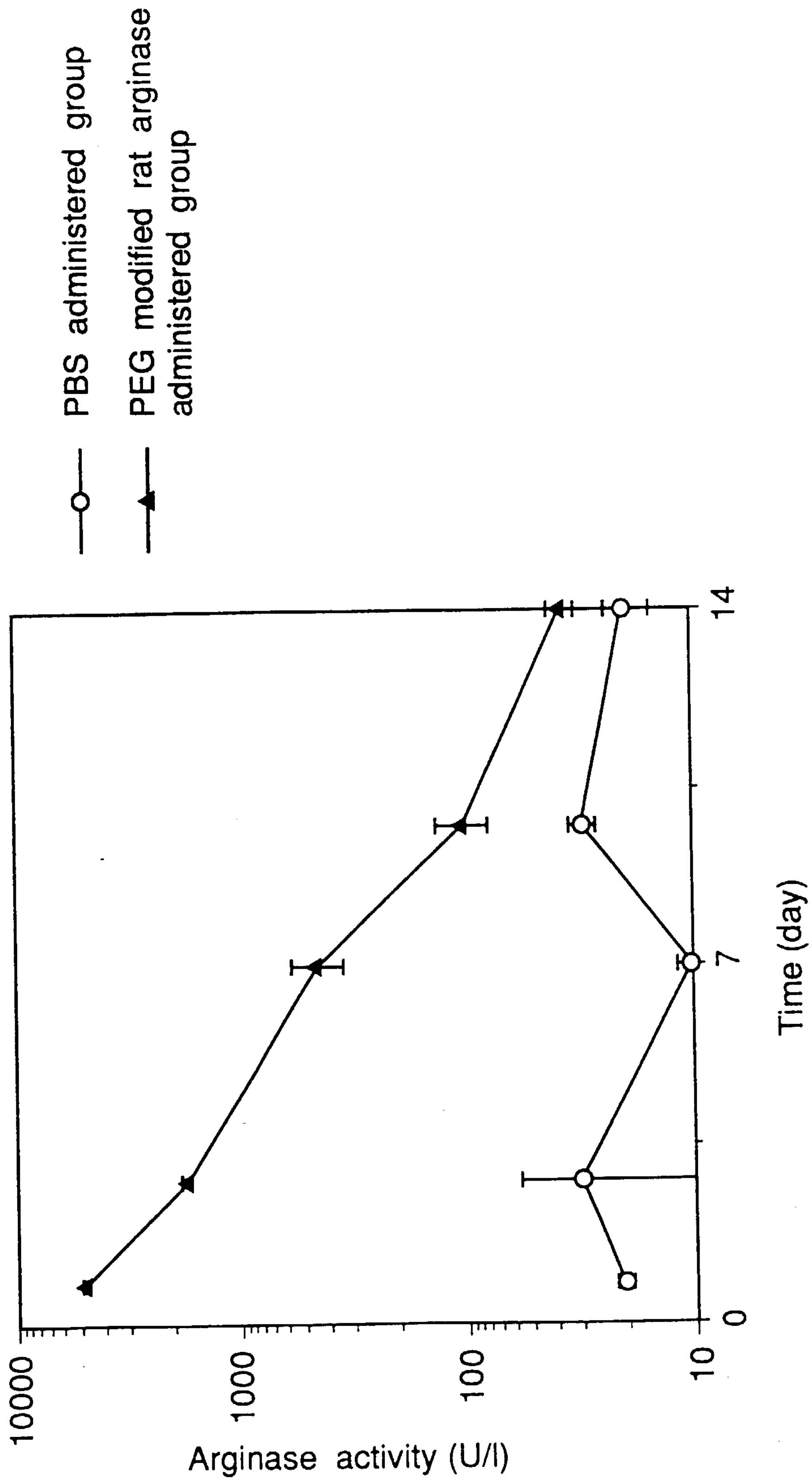
PBS administered group
PEG modified rat arginase administered group
Arginase activity (U/l)
10000
1000
100
10
Time (day)
0
7
14

## INTERNATIONAL SEARCH REPORT

International application No.
PCT/JP97/04423

**A. CLASSIFICATION OF SUBJECT MATTER**

Int. $Cl^6$ A61K38/50, C12N9/80, C12N15/55

According to International Patent Classification (IPC) or to both national classification and IPC

**B. FIELDS SEARCHED**

Minimum documentation searched (classification system followed by classification symbols)

Int. $Cl^6$ A61K38/50, C12N9/80, C12N15/55

Documentation searched other than minimum documentation to the extent that such documents are included in the fields searched

Electronic data base consulted during the international search (name of data base and, where practicable, search terms used)

Medline, Biosis Previews, GenBank

**C. DOCUMENTS CONSIDERED TO BE RELEVANT**

| Category* | Citation of document, with indication, where appropriate, of the relevant passages | Relevant to claim No. |
|---|---|---|
| Y | Arch. Pathol. Lab. Med., Vol. 111, (1987), Zimmermann A. et al. "Liver Fibrosis In Arginase Deficiency" p. 691-692 | 1 - 7 |
| Y | Am. J. Hum. Genet., Vol. 51, (1992), Uchino T. et al. "Three Novel Mutation in the Liver-Type Argonase Gene in Three Unrelated Japanese Patients with Arginemia" p. 1406-1412 | 1 - 7 |
| X | Proc. Natl. Acad. Sci. USA., Vol. 84, (1987), Haraguchi Y. et al. "Molecular cloning and nucleotide sequence of cDNA for human liver arginase" p. 412-415 | 8 - 12 |
| X<br>A | The Journal of Biological Chemistry, Vol. 262, (1987), Kawamoto S. et al. "Complete Nucleotide Sequence of cDNA and Deduced Amino Acid Sequence of Rat Liver Arginase" p. 6280-6283 | 13 - 17<br>1 - 7 |
| A | JP, 61-165335, A (Fumio Kuzuya), July 26, 1986 (26. 07. 86)(Family: none) | 1 - 7 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| January 29, 1998 (29. 01. 98) | February 10, 1998 (10. 02. 98) |
| Name and mailing address of the ISA/<br>Japanese Patent Office<br>Facsimile No. | Authorized officer<br><br>Telephone No. |

Form PCT/ISA/210 (second sheet) (July 1992)

**INTERNATIONAL SEARCH REPORT**

International application No.
PCT/JP97/04423

C (Continuation). DOCUMENTS CONSIDERED TO BE RELEVANT

| Category* | Citation of document, with indication, where appropriate, of the relevant passages | Relevant to claim No. |
|---|---|---|
| A | JP, 2-35081, A (Agency of Industrial Science and Technology), February 5, 1990 (05. 02. 90)(Family: none) | 1 - 7 |
| A | JP, 3-164173, A (Japan Energy Corp.), July 16, 1991 (16. 07. 91) & EP, 414007, A & US, 5474928, A | 1 - 7 |
| A | JP, 4-121187, A (Japan Energy Corp.), April 22, 1992 (22. 04. 92)(Family: none) | 1 - 7 |
| A | US, 5196195, A (Cornell Research Foundation Inc.), March 23, 1993 (23. 03. 93)(Family: none) | 1 - 7 |

Form PCT/ISA/210 (continuation of second sheet) (July 1992)